# EUROPEAN PATENT APPLICATION

(11) **EP 3 916 102 A1**
(43) Date of publication of application: **01.12.2021**
(21) Application number: 20770845.4
(22) Date of filing: 04.03.2020
(51) Int. Cl.: C12P 19/02

(54) **ALLULOSE-PRODUCING MICROORGANISM OF GENUS STAPHYLOCOCCUS AND METHOD FOR MANUFACTURING ALLULOSE USING SAME**

(30) Priority: 08.03.2019 KR 20190026633
(71) Applicant: CJ Cheiljedang Corporation, Seoul 04560 (KR)
(72) Inventor: KIM, Su Jin, Seoul 04560 (KR); CHI, Hyun, Seoul 04560 (KR); HONG, Eunsoo, Seoul 04560 (KR); KIM, Yang Hee, Seoul 04560 (KR); KIM, Taek Beom, Seoul 04560 (KR); GWAK, Junseok, Seoul 04560 (KR); KIM, Seong Bo, Seoul 04560 (KR); CHOI, Eun Jung, Seoul 04560 (KR)
(74) Representative: Regimbeau
(86) International application number: PCT/KR2020/003061
(87) International publication number: WO 2020/184889

(57) **Abstract**

The present application relates to a microorganism that produces allulose and a method for preparing allulose using the same.

## Description

### [Technical Field]

The present application relates to a microorganism that produces allulose and a method for preparing allulose using the same.

### [Background Art]

D-Allulose is a C3 epimer of D-fructose and is a monosaccharide known as a rare sugar that is present in a significantly small amount in nature. The sweetness of D-allulose is about 70% of the sweetness of sugar, but the caloric value thereof is almost zero. D-Allulose has functions of suppressing the elevation of blood sugar levels, lipid synthesis, and the like, and thus it is receiving much attention as a new food ingredient that can be used in functional foods.

Due to these properties, allulose is considered for use in various foods as a sugar substitute sweetener. However, allulose is present in a significantly small amount in nature, and there is thus a growing need for a method by which allulose can be efficiently manufactured.

As the conventionally known production method of allulose, a chemical method has been known in which allulose is produced from D-fructose by using the catalytic action of molybdate ions or by heating D-fructose with ethanol and triethylamine. Such a chemical method has disadvantages such as a low allulose production yield and high manufacturing cost.

In order to solve such problems, a biological method for producing allulose from fructose using an epimerase derived from microorganisms has been studied. Starting with a technology for producing allulose using genetically modified microorganisms with a D-allulose 3-epimerization gene derived from *Agrobacterium tumefaciens,* a method for producing allulose from D-fructose by separating microbial strains from the natural environment or food and using these microbial strains has also been recently reported (Korean Patent Publication No. 10-2011-0035805, Korean Patent Publication No. 10-1804778, Korean Patent Publication No. 10-2017-0067070, and Korean Patent Publication No. 10-2016-00817220). Biological technologies for producing allulose have been developed, but there is an urgent need to discover new microbial resources that can produce allulose because of the low thermal stability of microorganisms used as biocatalysts or high production cost.

With this background, as a result of diligent studies to develop microorganisms that can produce allulose even in a high-temperature environment, it has been confirmed that microorganisms belonging to the genus *Staphylococcus* can produce allulose, and thus the present application has been completed.

### [Disclosure]

### [Technical Problem]

The present application provides a composition for allulose production comprising a microorganism belonging to the genus *Staphylococcus.*

The present application provides a method for preparing allulose by using the composition.

### [Technical Solution]

The technical solution will be described in detail as follows. Meanwhile, each description and embodiment disclosed in the present application may also be applied to other descriptions and embodiments. That is, all combinations of various elements disclosed in the present application fall within the scope of the present application. Further, the scope of the present application is not limited by the specific description below.

An aspect of the present application may provide a composition for allulose production comprising a microorganism belonging to the genus *Staphylococcus* or a culture of the microorganism belonging to the genus *Staphylococcus.*

Specifically, the microorganism belonging to the genus *Staphylococcus* may be any one selected from the group consisting of *Staphylococcus agnetis, Staphylococcus argensis, Staphylococcus argenteus, Staphylococcus arlettae, Staphylococcus aureus, Staphylococcus auricularis, Staphylococcus capitis, Staphylococcus caprae, Staphylococcus carnosus, Staphylococcus chromogenes, Staphylococcus cohnii, Staphylococcus condiment, Staphylococcus cornubiensis, Staphylococcus delphini, Staphylococcus devriesei, Staphylococcus edaphicus, Staphylococcus epidermidis, Staphylococcus equorum, Staphylococcus faecalis, Staphylococcus felis, Staphylococcus fleurettii, Staphylococcus gallinarum, Staphylococcus haemolyticus, Staphylococcus hominis, Staphylococcus hyicus, Staphylococcus intermedius, Staphylococcus kloosii, Staphylococcus leei, Staphylococcus lentus, Staphylococcus lugdunensis, Staphylococcus lutrae, Staphylococcus lyticans, Staphylococcus massiliensis, Staphylococcus microti, Staphylococcus muscae, Staphylococcus nepalensis, Staphylococcus pasteuri, Staphylococcus petrasii, Staphylococcus pettenkoferi, Staphylococcus piscifermentans, Staphylococcus pseudintermedius, Staphylococcus pseudolugdunensis, Staphylococcus rostri, Staphylococcus saccharolyticus, Staphylococcus saprophyticus, Staphylococcus schleiferi, Staphylococcus schweitzeri, Staphylococcus sciuri, Staphylococcus simiae, Staphylococcus simulans, Staphylococcus stepanovicii, Staphylococcus succinus, Staphylococcus vitulinus, Staphylococcus warneri,* and *Staphylococcus xylosus,* but is not limited thereto.

The microorganism belonging to the genus *Staphylococcus* may be more specifically any one selected from the group consisting of *Staphylococcus carnosus, Staphylococcus xylosus, Staphylococcus vitulinus, Staphylococcus epidermidis, Staphylococcus warneri, Staphylococcus haemolyticus, Staphylococcus intermedius, Staphylococcus saprophyticus, Staphylococcus cohnii, Staphylococcus muscae, Staphylococcus lentus, Staphylococcus chromogenes, Staphylococcus caprae, Staphylococcus auricularis, Staphylococcus gallinarum, Staphylococcus arlettae, Staphylococcus equorum, Staphylococcus kloosii, Staphylococcus delphini, and Staphylococcus pasteuri,* still more specifically *Staphylococcus carnosus, Staphylococcus xylosus,* or *Staphylococcus vitulinus,* but is not limited thereto.

In the present application, D-allulose (hereinafter referred to as allulose) is an epimer of D-fructose and is prepared from fructose by an epimerase. In the present application, D-allulose may be used interchangeably with psicose.

In the present application, the fructose used as a substrate may be obtained from sugar decomposed by a converting enzyme or from high-fructose corn syrup, or may be purchased as a commercial product, but is not limited thereto.

The microorganism belonging to the genus *Staphylococcus* of the present application has the activity of converting D-fructose to D-allulose. The microorganism belonging to the genus *Staphylococcus* produces D-allulose from D-fructose by using its metabolic system. Such a conversion reaction may be conducted within the microorganism or outside of the microorganism by way of the substances secreted, but a microorganism belonging to the genus *Staphylococcus* falls within the scope of the present application without limitation of the allulose producing process as long as it is a microorganism belonging to the genus *Staphylococcus* that can produce allulose from fructose.

Meanwhile, the microorganism belonging to the genus *Staphylococcus* of the present application may mean not only wild-type microorganisms but also mutant microorganisms including mutations that occur naturally or non-naturally. Specifically, mutations occurring non-naturally may be to mutate wild-type microorganisms or naturally mutated microorganisms using UV irradiation, radiation (gamma ray, X-ray), or chemical mutagens. A microorganism belonging to the genus *Staphylococcus* falls within the scope of the present application as long as it has the properties of the microorganism belonging to the genus *Staphylococcus* that has the ability to produce allulose from fructose even though the microorganism has a genetic trait different from that of a wild-type microorganism belonging to the genus *Staphylococcus.*

The microorganism belonging to the genus *Staphylococcus* of the present application exhibits heat resistance so as to be able to produce allulose even in a high-temperature environment, for example, at a temperature of 50°C or more, and thus has an advantage of increasing the yield of allulose production.

The microorganism belonging to the genus *Staphylococcus* of the present application exhibits the activity of converting fructose to allulose, and a microorganism belonging to the genus *Staphylococcus* may fall within the scope of the present application as long as it has an allulose conversion rate that can be industrially used.

In the present application, the conversion rate may be expressed as the concentration of allulose produced by 12 hours of reaction/initial fructose concentration (1 wt%), and the microorganism belonging to the genus *Staphylococcus* of the present application may have a conversion rate of 0.1% or more, specifically 0.3% or more, 0.5% or more, 0.9% or more, 1.6% or more, 2.3% or more, 3.4% or more, 5.3% or more, 10.4% or more, or 24.5% or more, but the conversion rate is not limited thereto.

In the present application, the conversion rate may be measured by way of a method known in the art, and the method is not limited to a specific method. As an example, in the present application, the result of conversion reaction to allulose conducted at a pH of 7.5 and 55°C for 12 hours was measured as the conversion rate, but the reaction conditions (for example, pH, temperature, time, and the like) may be appropriately selected by those skilled in the art to measure the conversion rate.

In particular, the microorganism belonging to the genus *Staphylococcus* is known not to have pathogenicity and thus has an advantage of being able to be used in various foods.

Accordingly, the microorganism belonging to the genus *Staphylococcus* of the present application may be nonpathogenic, but is not limited thereto. Specifically, the microorganism belonging to the genus *Staphylococcus* that is known as a nonpathogenic microorganism may be *Staphylococcus argensis, Staphylococcus capitis, Staphylococcus devriesei, Staphylococcus faecalis, Staphylococcus sciuri, Staphylococcus hominis, Staphylococcus lugdunensis, Staphylococcus microti, Staphylococcus piscifermentans, Staphylococcus schweitzeri, Staphylococcus simulans, Staphylococcus succinus, Staphylococcus arlettae, Staphylococcus auricularis, Staphylococcus caprae, Staphylococcus carnosus, Staphylococcus chromogenes, Staphylococcus cohnii, Staphylococcus delphini, Staphylococcus epidermidis, Staphylococcus equorum, Staphylococcus gallinarum, Staphylococcus haemolyticus, Staphylococcus intermedius, Staphylococcus kloosii, Staphylococcus lentus, Staphylococcus muscae, Staphylococcus pasteuri, Staphylococcus saprophyticus, Staphylococcus vitulinus, Staphylococcus warneri,* or *Staphylococcusxylosus,* but is not limited thereto.

In the present application, nonpathogenic microorganisms mean microorganisms that do not bring symptoms of disease in individuals including humans, and mean safe strains corresponding to the internationally accepted biosafety level 1. Specifically, the nonpathogenic microorganisms mean all strains except *Staphylococcus agnetis, Staphylococcus argenteus, Staphylococcus aureus, Staphylococcus cornubiensis, Staphylococcus felis, Staphylococcus fleurettii, Staphylococcus hyicus, Staphylococcus lutrae, Staphylococcus massiliensis, Staphylococcus nepalensis, Staphylococcus petrasii, Staphylococcus pettenkoferi, Staphylococcus pseudintermedius, Staphylococcus rostri, Staphylococcus schleiferi, Staphylococcus simiae,* and *Staphylococcus stepanovicii* strains that correspond to the biosafety level 2 or more among the microorganisms belonging to the genus *Staphylococcus.*

The nonpathogenic microorganism belonging to the genus *Staphylococcus* of the present application does not adversely affect individuals including humans while having the ability to produce allulose.

In the present application, it has been confirmed that various microorganisms belonging to the genus *Staphylococcus,* in particular, nonpathogenic microorganisms, not only have the activity of converting fructose to allulose but also have genetic connectivity therebetween through the analysis of 16S rRNA of the microorganisms.

In the present application, "16S rRNA" refers to 16S ribosomal RNA, the rRNA component of the 30S subunit of a prokaryotic ribosome, having a length of about 1,500 nucleotides. The 16S rRNA sequence is known to be a sequence generally used to identify prokaryotes since the 16S rRNA sequence is most highly conserved while exhibiting high base sequence diversity in some sections and, in particular, diversity rarely appears in the same species, whereas diversity appears between different species. In other words, the genetic connectivity may be determined by comparing the homology of the 16S rRNA sequence. It may be understood that prokaryotes have more similar genetic traits as the homology (similarity) of 16S rRNA sequence becomes higher. In the present application, the term "homology" or "identity" means the degree to which two given base sequences are related to each other, and may be expressed as a percentage.

The terms homology and identity may often be used interchangeably.

Conserved polynucleotide sequence homology or identity is determined by standard sequence alignment algorithms, and the default gap penalty established by the program being used may be used together. Substantially, homologous or identical sequences are capable of hybridizing generally to the full sequence or to at least about 50%, 60%, 70%, 80%, or 90% or more of the full length in moderate or highly stringent conditions. In hybridization, polynucleotides containing degenerate codons instead of codons in the polynucleotide are also contemplated.

Whether any two polynucleotide sequences have homology, similarity, or identity may be determined, for example, using default parameters as in Pearson et al. (1988) Proc. Natl. Acad. Sci. USA 85:2444 and known computer algorithms such as the "FASTA" program. Alternatively, whether any two polynucleotide sequences have homology, similarity, or identity may be determined using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later). The GCG program package (Devereux, J. et al, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994; and CARILLO et al. (1988) SIAM J Applied Math 48:1073 are also included. For example, BLAST of the National Center for Biotechnology Information, the Clustal omega program (https://www.ebi.ac.uk/Tools/msa/clustalo), or ClustalW may be used to determine homology, similarity, or identity.

The homology, similarity or identity of polynucleotides may be determined by comparing the sequence information thereof, for example, using the GAP computer program such as Needleman et al. (1970), J Mol Biol. 48:443, for example, as known in Smith and Waterman, Adv. Appl. Math (1981) 2:482. In short, the homology, similarity, or identity of polynucleotides is defined as the value obtained by dividing the number of similarly aligned symbols (namely, nucleotides or amino acids) by the total number of symbols in the shorter of the two sequences by the GAP program. The default parameters for the GAP program may include (1) a unary numeral system comparison matrix (containing a value of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for an end gap. Hence, as used in the present application, the term "homology" or "identity" refers to relatedness between sequences.

The composition for allulose production of the present application may comprise one, two, or more microorganism(s) belonging to the genus *Staphylococcus* or a culture thereof, but is not limited thereto.

The composition for allulose production of the present application comprises a microorganism belonging to the genus *Staphylococcus* that exhibits the activity of converting fructose to allulose or a culture thereof and thus can produce allulose.

In the present application, "culturing" means to grow a microorganism under appropriate artificially controlled environmental conditions, and "culture" means a product obtained by culturing a microorganism and may contain a microorganism or all substances secreted from the microorganism.

In the present application, culturing of a microorganism belonging to the genus *Staphylococcus* may be performed by way of a method widely known in the art. Specifically, the culturing may be performed batchwise, continuously, or fedbatchwise in a batch, injection and batch, or repeated injection and batch process, but is not limited thereto.

The medium used for culturing must meet the requirements for a specific strain in an appropriate manner, and the medium conditions for culturing of microorganisms belonging to the genus *Staphylococcus* are known.

Specifically, the sugar source that may be used in the medium includes sugars and carbohydrates such as glucose, saccharose, lactose, fructose, maltose, starch, and cellulose, oils and fats such as soybean oil, sunflower oil, castor oil, and coconut oil, fatty acids such as palmitic acid, stearic acid, and linoleic acid, alcohols such as glycerol and ethanol, and organic acids such as acetic acid. These substances may be used individually or as a mixture, but the way to use the sugar sources is not limited thereto.

The carbon source that may be used may be raw sugar or glucose, molasses containing a large amount of raw sugar, and specifically purified glucose, but is not limited thereto, and other carbon sources may be used in various ways.

The nitrogen source that may be used includes peptone, yeast extract, broth, malt extract, corn steep liquor, soybean meal, and urea or inorganic compounds such as ammonium sulfate, ammonium chloride, ammonium phosphate, ammonium carbonate, and ammonium nitrate. The nitrogen sources may also be used individually or as a mixture, but the way to use the nitrogen sources is not limited thereto.

The phosphorus source that may be used may include potassium dihydrogen phosphate or dipotassium hydrogen phosphate or the corresponding salt containing sodium.

The culture medium may contain a metal salt such as magnesium sulfate or iron sulfate required for growth. In addition to the substances, essential growth substances such as amino acids and vitamins may be used. Precursors suitable for the culture medium may be used. The above-described raw materials may be added batchwise or continuously in a manner suitable for the culture during the culturing process.

The pH of the culture may be adjusted during the culturing of a microorganism using basic compounds such as sodium hydroxide, potassium hydroxide, and ammonia or acidic compounds such as phosphoric acid or sulfuric acid in an appropriate manner. Foaming may be suppressed using an antifoaming agent such as fatty acid polyglycol ester. Oxygen or an oxygen-containing gas (for example, air) may be injected into the culture to maintain the aerobic condition.

The composition for allulose production of the present application may further comprise fructose, which is a substrate and/or an enzyme involved in the allulose production, in addition to the microorganism belonging to the genus *Staphylococcus* or a culture of the microorganism, but is not limited thereto.

The composition for allulose production of the present application may further contain arbitrary suitable excipients that are commonly used in the composition for allulose production. Such excipients may include, for example, a preservative, a wetting agent, a dispersing agent, a suspending agent, a buffering agent, a stabilizing agent, and an isotonizing agent, but are not limited thereto.

The composition for allulose production of the present application may further contain a metal ion or metal salt. The composition for allulose production of the present application may exhibit the activity of converting fructose to allulose since the metal ion or metal salt is contained in the composition. The metal ion or metal salt may be required in the process of producing allulose from fructose by a microorganism belonging to the genus *Staphylococcus,* for example, it may be required by the action of an enzyme that mediates the conversion process, but is not limited thereto. As for the metal ion or metal salt contained in the composition for allulose production of the present application, a metal ion or metal salt known to those skilled in the art may be appropriately selected as long as the composition can exhibit the activity of converting fructose to allulose. In an embodiment, the metal ion may be a divalent cation, specifically ions of one or more metals selected from the group consisting of Ni, Mg, Ni, Co, Mn, Fe, and Zn. More specifically, the composition for allulose production of the present application may further contain a metal salt. Still more specifically, the metal salt may be one or more selected from the group consisting of NiSO₄, MgSO₄, MgCl₂, NiCl₂, CoSO₄, CoCl₂, MnCl₂, MnSO₄, FeSO₄, and ZnSO₄.

Another aspect of the present application may provide the use of a microorganism belonging to the genus *Staphylococcus* or a culture of the microorganism belonging to the genus *Staphylococcus* for allulose production.

The "microorganism belonging to the genus *Staphylococcus"* and "culture of a microorganism belonging to the genus *Staphylococcus"* are as described above.

Still another aspect of the present application provides a method for preparing allulose, which includes bringing the composition into contact with fructose to convert the fructose to allulose.

Specifically, the microorganism belonging to the genus *Staphylococcus* may be any one selected from the group consisting of *Staphylococcus agnetis, Staphylococcus argensis, Staphylococcus argenteus, Staphylococcus arlettae, Staphylococcus aureus, Staphylococcus auricularis, Staphylococcus capitis, Staphylococcus caprae, Staphylococcus carnosus, Staphylococcus chromogenes, Staphylococcus cohnii, Staphylococcus condiment, Staphylococcus cornubiensis, Staphylococcus delphini, Staphylococcus devriesei, Staphylococcus edaphicus, Staphylococcus epidermidis, Staphylococcus equorum, Staphylococcus faecalis, Staphylococcus felis, Staphylococcus fleurettii, Staphylococcus gallinarum, Staphylococcus haemolyticus, Staphylococcus hominis, Staphylococcus hyicus, Staphylococcus intermedius, Staphylococcus kloosii, Staphylococcus leei, Staphylococcus lentus, Staphylococcus lugdunensis, Staphylococcus lutrae, Staphylococcus lyticans, Staphylococcus massiliensis, Staphylococcus microti, Staphylococcus muscae, Staphylococcus nepalensis, Staphylococcus pasteuri, Staphylococcus petrasii, Staphylococcus pettenkoferi, Staphylococcus piscifermentans, Staphylococcus pseudintermedius, Staphylococcus pseudolugdunensis, Staphylococcus rostri, Staphylococcus saccharolyticus, Staphylococcus saprophyticus, Staphylococcus schleiferi, Staphylococcus schweitzeri, Staphylococcus sciuri, Staphylococcus simiae, Staphylococcus simulans, Staphylococcus stepanovicii, Staphylococcus succinus, Staphylococcus vitulinus, Staphylococcus warneri,* and *Staphylococcus xylosus,* but is not limited thereto.

The microorganism belonging to the genus *Staphylococcus* may be more specifically any one selected from the group consisting of *Staphylococcus carnosus, Staphylococcus xylosus, Staphylococcus vitulinus, Staphylococcus epidermidis, Staphylococcus warneri, Staphylococcus haemolyticus, Staphylococcus intermedius, Staphylococcus saprophyticus, Staphylococcus cohnii, Staphylococcus muscae, Staphylococcus lentus, Staphylococcus chromogenes, Staphylococcus caprae, Staphylococcus auricularis, Staphylococcus gallinarum, Staphylococcus arlettae, Staphylococcus equorum, Staphylococcus kloosii, Staphylococcus delphini,* and *Staphylococcus pasteuri,* still more specifically *Staphylococcus carnosus, Staphylococcus xylosus,* or *Staphylococcus vitulinus,* but is not limited thereto.

The preparation method of the present application may additionally include obtaining fructose from sugar and glucose, but is not limited thereto. The method for obtaining fructose from sugar and glucose, in particular, a production method using an enzyme, is known in the art.

The preparation method of the present application has an advantage of discharging fewer pollutants and increasing the yield since fructose can be converted to allulose even in a high-temperature environment by this preparation method.

Specifically, the temperature in the converting fructose to allulose may be 40°C to 70°C, specifically 50°C to 70°C. The culturing may be continuously performed until a desired amount of allulose is produced, and the culturing time may be specifically 5 to 120 hours, more specifically 10 to 30 hours, but is not limited thereto. In the preparation method of the present application, conversion of fructose to allulose may be performed at a pH of 5.0 to 9.0, specifically a pH of 6.0 to 8.0.

The preparation method of the present application may further include recovering allulose converted by a microorganism belonging to the genus *Staphylococcus,* but is not limited thereto.

Specifically, allulose may be recovered by crushing the microorganism belonging to the genus *Staphylococcus,* or allulose may be separated from the culture of the microorganism belonging to the genus *Staphylococcus,* but the recovery method is not limited to a specific method as long as allulose converted by the microorganism belonging to the genus *Staphylococcus* can be recovered.

Separation of allulose may be performed by way of conventional methods known in the art. As such a separation method, methods such as centrifugation, filtration, ion-exchange chromatography, and crystallization may be used. For example, the culture may be centrifuged at a low speed to remove biomass, and the obtained supernatant may be subjected to ion-exchange chromatography for separation of allulose, but the separation method is not limited thereto.

The preparation method of the present application may further include purifying allulose, but is not limited thereto. The purification may be performed by way of a conventionally used method, and non-limiting examples thereof may include dialysis, precipitation, adsorption, electrophoresis, ion-exchange chromatography, and fractional crystallization. The purification may be performed by way of only one method or the combination of two or more methods. For example, the reaction mixture having produced allulose may be purified through chromatography, and the separation of sugar by chromatography may be performed using a difference in weak binding strength between the sugar to be separated and the metal ion attached to the ion-exchange resin.

The preparation method of the present application may further include performing decolorization or desalting or both decolorization and desalting before or after the allulose purifying step of the present application. By performing the decolorization and/or desalting, a more purified reaction mixture containing allulose without impurities may be obtained.

### [Advantageous Effects]

The microorganism belonging to the genus *Staphylococcus* of the present application has an effect of producing allulose even in a high-temperature environment.

### [Brief Description of Drawings]

FIG. 1 is a diagram for confirming the production of allulose by *Staphylococcus delphini* KCTC3592;
FIG. 2 is a diagram illustrating the phylogenetic tree of nonpathogenic *Staphylococcus* strains; and

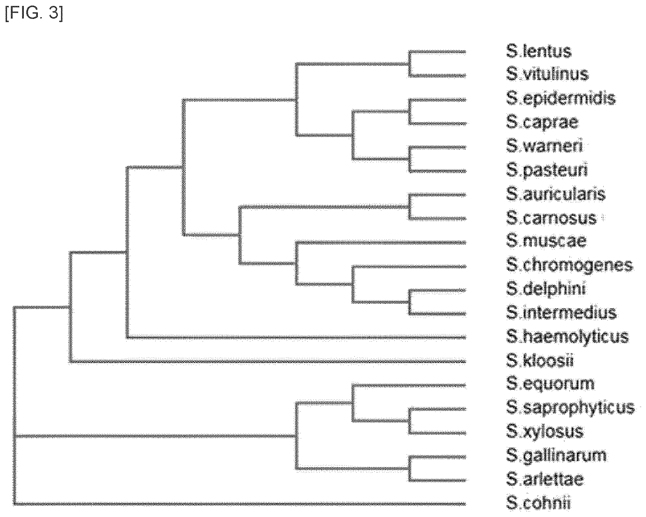

FIG. 3 is a diagram illustrating the phylogenetic tree of 20 nonpathogenic *Staphylococcus* strains.

### [Detailed Description of the Invention]

The best mode for carrying out the invention will be described in detail as follows. Meanwhile, each description and embodiment disclosed in the present application may also be applied to other descriptions and embodiments. That is, all combinations of various elements disclosed in the present application fall within the scope of the present application. Further, the scope of the present application is not limited by the specific description below.

In order to achieve the above object, the present application provides a composition for allulose production comprising a microorganism belonging to the genus *Staphylococcus* or a culture of the microorganism belonging to the genus *Staphylococcus.*

### Examples

Hereinafter, the present application will be described in more detail with reference to Examples. However, these Examples are for illustrative purposes only, and the scope of the present application is not limited to these Examples.

### Example 1: Confirmation of allulose production by microorganism belonging to genus Staphylococcus

In order to confirm the ability of microorganisms belonging to the genus *Staphylococcus* to produce allulose, 32 nonpathogenic microorganisms belonging to the genus *Staphylococcus* were selected, and it was confirmed whether 20 microorganisms belonging to the genus *Staphylococcus* among these produced allulose from D-fructose.

Specifically, 2 species of *Staphylococcus carnosus* (KCTC3580, KACC13250), 2 species of *Staphylococcus xylosus* (KCTC3342, KACC16180), 4 species of *Staphylococcus vitulinus* (KACC15803, KACC15804, KACC15805, KACC13211), *Staphylococcus delphini* (KCTC3592), *Staphylococcus equorum* (KCTC3589), *Staphylococcus epidermidis* (KCTC1917), *Staphylococcus cohnii* (KCTC3574), *Staphylococcus chromogenes* (KCTC3579), *Staphylococcus caprae* (KCTC3583), *Staphylococcus warneri* (KCTC3340), *Staphylococcus lentus* (KCTC3577), *Staphylococcus muscae* (KCTC3576), *Staphylococcus saprophyticus* (KCTC3345), *Staphylococcus pasteuri* (KCTC13167), *Staphylococcus intermedius* (KCTC3344), *Staphylococcus arlettae* (KCTC3588), *Staphylococcus kloosii* (KCTC3590), *Staphylococcus haemolyticus* (KCTC3341), *Staphylococcus gallinarum* (KCTC3585), and *Staphylococcus auricularis* (KCTC3584) were distributed from the Korean Collection for Type Cultures (KTCT) and the Korean Agricultural Culture Collection (KACC).

Each of the microorganisms was inoculated into Tryptic Soy Broth (peptone 17 g/L, soytone 3 g/L, psicose 10 g/L, NaCl 5 g/L, K₂HPO₄ 2.5 g/L, agar 15 g/L) to which allulose was added at 1%, and cultured at 30°C or 37°C for 18 hours. Thereafter, the cultured microorganisms were recovered, washed with 0.85% (w/v) NaCl, and then used to perform the whole-cell conversion reaction.

To the microorganism at a concentration of 20% (w/w), 50 mM potassium phosphate buffer (pH 7.5) to which D-fructose was added at 1% (w/w) was added and suspended, and the suspension was subjected to the conversion reaction at 55°C for 12 hours.

The supernatant of the conversion reaction product was subjected to HPLC analysis to confirm the allulose production. HPLC analysis was performed at a temperature of 80°C and a flow velocity of 0.6 mL/min using water as a mobile phase solvent and a Refractive Index Detector (Agilent 1260 RID) of HPLC (Agilent, USA) equipped with Aminex HPX-87C column (BIO-RAD). The allulose conversion rate was calculated as the ratio of the weight of allulose produced after the reaction to the weight of substrate (D-fructose) before the reaction (allulose concentration (12 h reaction)/initial fructose concentration (1 wt%)).

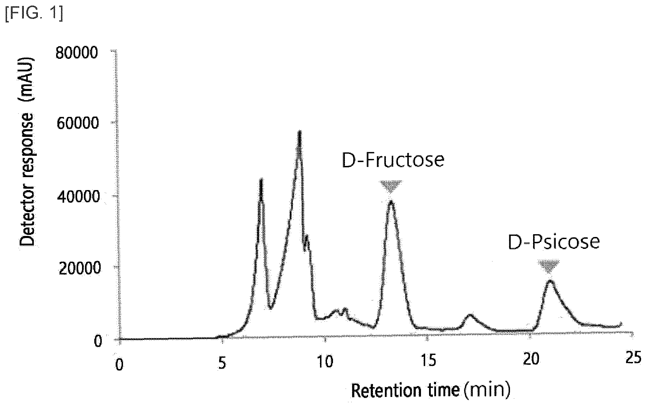

As a result, it has been confirmed that the 20 microorganisms belonging to the genus *Staphylococcus* all produce allulose from D-fructose (Table 1 and FIG. 1).

**[Table 1]**

| Name of strain | Conversion rate (%) |
|---|---|
| *Staphylococcus delphini* KCTC3592 | 29.8 |
| *Staphylococcus carnosus* KCTC3580 | 26.2 |
| *Staphylococcus carnosus* KACC13250 | 25.7 |
| *Staphylococcus equorum* KCTC3589 | 24.5 |
| *Staphylococcus epidermidis* KCTC1917 | 15.8 |
| *Staphylococcus cohnii* KCTC3574 | 10.4 |
| *Staphylococcus xylosus* KCTC3342 | 8.0 |
| *Staphylococcus xylosus* KACC16180 | 8.0 |
| *Staphylococcus chromogenes* KCTC3579 | 7.8 |
| *Staphylococcus caprae* KCTC3583 | 5.3 |
| *Staphylococcus vitulinus* KAC C15803 | 3.3 |
| *Staphylococcus vitulinus* KAC C15804 | 3.4 |
| *Staphylococcus warneri* KCTC3340 | 2.7 |
| *Staphylococcus lentus* KCTC3577 | 2.9 |
| *Staphylococcus vitulinus* KAC C15805 | 2.6 |
| *Staphylococcus muscae* KCTC3576 | 2.3 |
| *Staphylococcus pasteuri* KCTC13167 | 1.8 |
| *Staphylococcus intermedius* KCTC3344 | 1.7 |
| *Staphylococcus saprophyticus* KCTC3345 | 1.6 |
| *Staphylococcus arlettae* KCTC3588 | 1.2 |
| *Staphylococcus vitulinus* KAC C13211 | 0.9 |
| *Staphylococcus kloosii* KCTC3590 | 0.8 |
| *Staphylococcus haemolyticus* KCTC3341 | 0.5 |
| *Staphylococcus gallinarum* KCTC3585 | 0.4 |
| *Staphylococcus auricularis* KCTC3584 | 0.3 |

### Example 2: Confirmation of 16S rRNA similarity of microorganisms belonging to genus Staphylococcus

### Example 2-1: Confirmation of 16S rRNA similarity of nonpathogenic microorganisms belonging to genus Staphylococcus

Microorganisms classified as nonpathogenic (biosafety level 1) microorganisms belonging to the genus *Staphylococcus* in the present Example are as follows: *Staphylococcus argensis, Staphylococcus capitis, Staphylococcus devriesei, Staphylococcus faecalis, Staphylococcus sciuri, Staphylococcus hominis, Staphylococcus lugdunensis, Staphylococcus microti, Staphylococcus piscifermentans, Staphylococcus schweitzeri, Staphylococcus simulans, Staphylococcus succinus, Staphylococcus arlettae, Staphylococcus auricularis, Staphylococcus caprae, Staphylococcus carnosus, Staphylococcus chromogenes, Staphylococcus cohnii, Staphylococcus delphini, Staphylococcus epidermidis, Staphylococcus equorum, Staphylococcus gallinarum, Staphylococcus haemolyticus, Staphylococcus intermedius, Staphylococcus kloosii, Staphylococcus lentus, Staphylococcus muscae, Staphylococcus pasteuri, Staphylococcus saprophyticus, Staphylococcus vitulinus, Staphylococcus warneri,* and *Staphylococcus xylosus.*

The sequence homology between strains was analyzed using the Clustal omega program (https://www.ebi.ac.uk/Tools/msa/clustalo) based on the 16s rRNA sequences of 31 microorganisms among the nonpathogenic microorganisms belonging to the genus *Staphylococcus* except *Staphylococcus faecalis,* of which the 16s rRNA sequence was not identified. The representative 16s rRNA sequences of the respective strains were confirmed from the NCBI database.

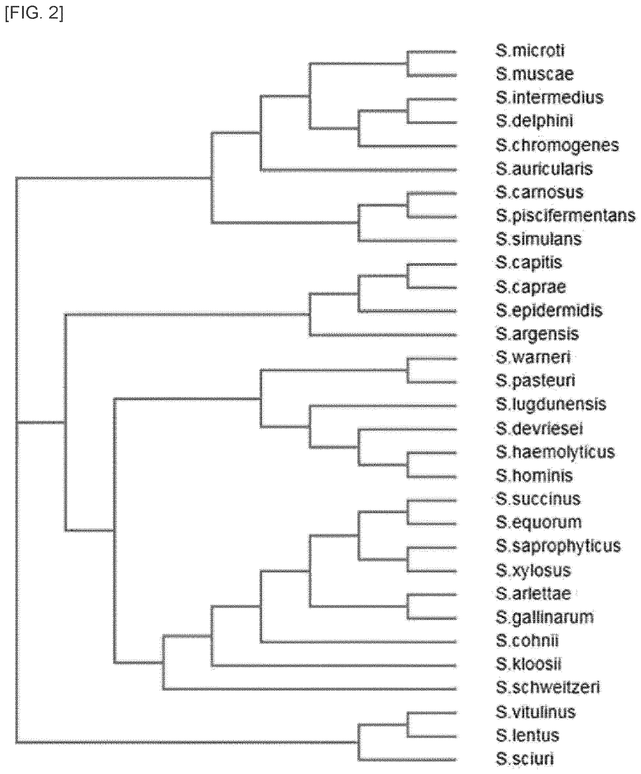

As a result, it has been confirmed that the homology of 16s rRNA sequences of the microorganisms belonging to the genus *Staphylococcus* known as nonpathogenic microorganisms between the strains is 92.78% or more in all cases (FIG. 2). The homology of the 31 microorganisms belonging to the genus *Staphylococcus* was in most cases 95% or more, and the homology was 92.78% to 95.21% in the case of *Staphylococcus argensis.*

This is a result supporting that the microorganisms belonging to the genus *Staphylococcus* classified as nonpathogenic microorganisms all have high genetic connectivity.

### Example 2-2: Confirmation of 16S rRNA similarity of 20 nonpathogenic microorganisms belonging to genus Staphylococcus

The sequence homology was analyzed using the Clustal omega program (https://www.ebi.ac.uk/Tools/msa/clustalo) based on the 16s rRNA sequences of the 20 microorganisms belonging to the genus *Staphylococcus* of which the ability to produce allulose was confirmed in Example 1 above. The representative 16s rRNA sequences of the respective strains were confirmed via the NCBI database.

As a result, it has been confirmed that the homology of 16s rRNA sequences of the 20 microorganisms belonging to the genus *Staphylococcus* of which the ability to produce allulose has been confirmed is 95.97% or more.

From the Examples above, it has been confirmed that nonpathogenic microorganisms belonging to the genus *Staphylococcus* all have high genetic connectivity between species, and the microorganisms belonging to the genus *Staphylococcus* that have such high genetic connectivity exhibit the activity of producing allulose from fructose.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims or equivalents of such metes and bounds are therefore intended to be embraced by the claims.

## Claims

1. A composition for allulose production comprising a microorganism belonging to the genus *Staphylococcus* or a culture of the microorganism belonging to the genus *Staphylococcus.*

2. The composition according to claim 1, wherein the microorganism belonging to the genus *Staphylococcus* is any one selected from the group consisting of *Staphylococcus agnetis, Staphylococcus argensis, Staphylococcus argenteus, Staphylococcus arlettae, Staphylococcus aureus, Staphylococcus auricularis, Staphylococcus capitis, Staphylococcus caprae, Staphylococcus carnosus, Staphylococcus chromogenes, Staphylococcus cohnii, Staphylococcus condiment, Staphylococcus cornubiensis, Staphylococcus delphini, Staphylococcus devriesei, Staphylococcus edaphicus, Staphylococcus epidermidis, Staphylococcus equorum, Staphylococcus faecalis, Staphylococcus felis, Staphylococcus fleurettii, Staphylococcus gallinarum, Staphylococcus haemolyticus, Staphylococcus hominis, Staphylococcus hyicus, Staphylococcus intermedius, Staphylococcus kloosii, Staphylococcus leei, Staphylococcus lentus, Staphylococcus lugdunensis, Staphylococcus lutrae, Staphylococcus lyticans, Staphylococcus massiliensis, Staphylococcus microti, Staphylococcus muscae, Staphylococcus nepalensis, Staphylococcus pasteuri, Staphylococcus petrasii, Staphylococcus pettenkoferi, Staphylococcus piscifermentans, Staphylococcus pseudintermedius, Staphylococcus pseudolugdunensis, Staphylococcus rostri, Staphylococcus saccharolyticus, Staphylococcus saprophyticus, Staphylococcus schleiferi, Staphylococcus schweitzeri, Staphylococcus sciuri, Staphylococcus simiae, Staphylococcus simulans, Staphylococcus stepanovicii, Staphylococcus succinus, Staphylococcus vitulinus, Staphylococcus warneri,* and *Staphylococcus xylosus.*

3. The composition according to claim 1, wherein the microorganism belonging to the genus *Staphylococcus* is any one selected from the group consisting of *Staphylococcus carnosus, Staphylococcus xylosus, Staphylococcus vitulinus, Staphylococcus epidermidis, Staphylococcus warneri, Staphylococcus haemolyticus, Staphylococcus intermedius, Staphylococcus saprophyticus, Staphylococcus cohnii, Staphylococcus muscae, Staphylococcus lentus, Staphylococcus chromogenes, Staphylococcus caprae, Staphylococcus auricularis, Staphylococcus gallinarum, Staphylococcus arlettae, Staphylococcus equorum, Staphylococcus kloosii, Staphylococcus delphini,* and *Staphylococcus pasteuri.*

4. The composition according to claim 1, wherein the microorganism belonging to the genus *Staphylococcus* is nonpathogenic.

5. A method for preparing allulose, the method comprising bringing a microorganism belonging to the genus *Staphylococcus* or a culture of the microorganism belonging to the genus *Staphylococcus* into contact with fructose to convert the fructose to allulose.

6. The preparation method according to claim 5, wherein the microorganism belonging to the genus *Staphylococcus* is any one selected from the group consisting of *Staphylococcus agnetis, Staphylococcus argensis, Staphylococcus argenteus, Staphylococcus arlettae, Staphylococcus aureus, Staphylococcus auricularis, Staphylococcus capitis, Staphylococcus caprae, Staphylococcus carnosus, Staphylococcus chromogenes, Staphylococcus cohnii, Staphylococcus condiment, Staphylococcus cornubiensis, Staphylococcus delphini, Staphylococcus devriesei, Staphylococcus edaphicus, Staphylococcus epidermidis, Staphylococcus equorum, Staphylococcus faecalis, Staphylococcus felis, Staphylococcus fleurettii, Staphylococcus gallinarum, Staphylococcus haemolyticus, Staphylococcus hominis, Staphylococcus hyicus, Staphylococcus intermedius, Staphylococcus kloosii, Staphylococcus leei, Staphylococcus lentus, Staphylococcus lugdunensis, Staphylococcus lutrae, Staphylococcus lyticans, Staphylococcus massiliensis, Staphylococcus microti, Staphylococcus muscae, Staphylococcus nepalensis, Staphylococcus pasteuri, Staphylococcus petrasii, Staphylococcus pettenkoferi, Staphylococcus piscifermentans, Staphylococcus pseudintermedius, Staphylococcus pseudolugdunensis, Staphylococcus rostri, Staphylococcus saccharolyticus, Staphylococcus saprophyticus, Staphylococcus schleiferi, Staphylococcus schweitzeri, Staphylococcus sciuri, Staphylococcus simiae, Staphylococcus simulans, Staphylococcus stepanovicii, Staphylococcus succinus, Staphylococcus vitulinus, Staphylococcus warneri,* and *Staphylococcus xylosus.*

7. The preparation method according to claim 5, wherein the microorganism belonging to the genus *Staphylococcus* is any one selected from the group consisting of *Staphylococcus carnosus, Staphylococcus xylosus, Staphylococcus vitulinus, Staphylococcus epidermidis, Staphylococcus warneri, Staphylococcus haemolyticus, Staphylococcus intermedius, Staphylococcus saprophyticus, Staphylococcus cohnii, Staphylococcus muscae, Staphylococcus lentus, Staphylococcus chromogenes, Staphylococcus caprae, Staphylococcus auricularis, Staphylococcus gallinarum, Staphylococcus arlettae, Staphylococcus equorum, Staphylococcus kloosii, Staphylococcus delphini,* and *Staphylococcus pasteuri.*

8. The preparation method according to claim 5, which further comprises recovering converted allulose.

9. The preparation method according to claim 5, wherein the converting fructose to allulose is performed at a temperature of 40°C to 70°C.

10. Use of a microorganism belonging to the genus *Staphylococcus* or a culture of the microorganism belonging to the genus *Staphylococcus* for allulose production.


<110>	CJ CheilJedang Corporation

<120>	Allulose-producing Staphylococcus microorganism and method for
	producing allulose using the same

<130>	OPA20030-PCT

<150>	KR 10-2019-0026633
<151>	2019-03-08

<160>	31

<170>	KoPatentIn 3.0

<210>	1
<211>	1477
<212>	RNA
<213>	Staphylococcus gallinarum

<400>	1
aggatgaacg ctggcggcgt gcctaataca tgcaagtcga gcgaacagat aaggagcttg	60

ctcctttgac gttagcggcg gacgggtgag taacacgtgg gtaacctacc tataagactg	120

gaataactcc gggaaaccgg ggctaatgcc ggataacata tagaaccgca tggttctata	180

gtgaaagatg gttttgctat cacttataga tggacccgcg ccgtattagc tagttggtaa	240

ggtaatggct taccaaggcg acgatacgta gccgacctga gagggtgatc ggccacactg	300

gaactgagac acggtccaga ctcctacggg aggcagcagt agggaatctt ccgcaatggg	360

cgaaagcctg acggagcaac gccgcgtgag tgatgaaggg tttcggctcg taaaactctg	420

ttattaggga agaacatatg tgtaagtaac tgtgcacatc ttgacggtac ctaatcagaa	480

agccacggct aactacgtgc cagcagccgc ggtaatacgt aggtggcaag cgttatccgg	540

aattattggg cgtaaagcgc gcgtaggcgg tttcttaagt ctgatgtgaa agcccacggc	600

tcaaccgtgg agggtcattg gaaactggga aacttgagtg cagaagagga aagtggaatt	660

ccatgtgtag cggtgaaatg cgcagagata tggaggaaca ccagtggcga aggcgacttt	720

ctggtctgta actgacgctg atgtgcgaaa gcgtggggat caaacaggat tagataccct	780

ggtagtccac gccgtaaacg atgagtgcta agtgttaggg ggtttccgcc ccttagtgct	840

gcagctaacg cattaagcac tccgcctggg gagtacgacc gcaaggttga aactcaaagg	900

aattgacggg gacccgcaca agcggtggag catgtggttt aattcgaagc aacgcgaaga	960

accttaccaa atcttgacat cctttgacca ctctagagat agagctttcc ccttcggggg	1020

acaaagtgac aggtggtgca tggttgtcgt cagctcgtgt cgtgagatgt tgggttaagt	1080

cccgcaacga gcgcaaccct taagcttagt tgccatcatt aagttgggca ctctaggttg	1140

actgccggtg acaaaccgga ggaaggtggg gatgacgtca aatcatcatg ccccttatga	1200

tttgggctac acacgtgcta caatggacaa tacaaagggc agctaaaccg cgaggtcatg	1260

caaatcccat aaagttgttc tcagttcgga ttgtagtctg caactcgact acatgaagct	1320

ggaatcgcta gtaatcgtag atcagcatgc tacggtgaat acgttcccgg gtcttgtaca 1380

caccgcccgt cacaccacga gagtttgtaa cacccgaagc cggtggagta accatttatg 1440

gagctagccg tcgaaggtgg gacaaatgat tggggtg 1477

<210> 2
<211> 1493
<212> RNA
<213> Staphylococcus delphini

<400> 2
agagtttgat cctggctcag tatgaacgct ggcggcgtgc ctaatacatg caagtcgagc 60

gaacagataa ggagcttgct cctttgacgt tagcggcgga cgggtgagta acacgtgggt 120

aacctaccta taagactgga ataactccgg gaaaccgggg ctaatgccgg ataacatgtt 180

gaaccgcatg gttctacagt gaaagacggt cttgctgtca cttatagatg gacccgcgcc 240

gtattagcta gttggtgggg taacggccta ccaaggcgac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

tcggatcgta aagctctgtt gttagggaag aacaaatgtg taagtaactg tgcacatctt 480

gacggtacct aaccagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactggaaaa cttgagtgca 660

gaagaggaaa gtggaattcc atgtgtagcg gtgaaatgcg cagagatatg gaggaacacc 720

agtggcgaag gcggctttct ggtctgcaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacggtcgc 900

aagactgaaa ctcaaaggaa ttgacgggga cccgcacaag cggtggagca tgtggtttaa 960

ttcgaagcaa cgcgaagaac cttaccaaat cttgacatcc tttgacaact ctagagatag 1020

agctttcctc ttcggaggac aaagtgacag gtggtgcatg gttgtcgtca gctcgtgtcg 1080

tgagatgttg ggttaagtcc cgcaacgagc gcaacccttg aacttagttg ccatcattaa 1140

gttgggcact ctaagttgac tgccggtgac aaaccggagg aaggtgggga tgacgtcaaa 1200

tcatcatgcc ccttatgatt tgggctacac acgtgctaca atggacaata caaagggcag 1260

cgaaaccgcg aggtcaagca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac cattttggag ctagccgtcg aaggtgggac aaatgattgg ggt 1493

<210>     3
<211>     1470
<212>     RNA
<213>     Staphylococcus warneri

<400>     3
gatgaacgct ggcggcgtgc ctaatacatg caagtcgagc gaacagataa ggagcttgct      60

cctttgacgt tagcggcgga cgggtgagta acacgtggat aacctaccta taagactggg     120

ataacttcgg gaaaccggag ctaataccgg ataacatatt gaaccgcatg gttcaatagt     180

gaaaggcggc tttgctgtca cttatagatg gatccgcgcc gtattagcta gttggtaagg     240

taacggctta ccaaggcaac gatacgtagc cgacctgaga gggtgatcgg ccacactgga     300

actgagacac ggtccagact cctacgggag gcagcagtag ggaatcttcc gcaatgggcg     360

aaagcctgac ggagcaacgc cgcgtgagtg atgaaggtct tcggatcgta aaactctgtt     420

atcagggaag aacaaatgtg taagtaactg tgcacatctt gacggtacct gatcagaaag     480

ccacggctaa ctacgtgcca gcagccgcgg taatacgtag gtggcaagcg ttatccggaa     540

ttattgggcg taaagcgcgc gtaggcggtt ttttaagtct gatgtgaaag cccacggctc     600

aaccgtggag ggtcattgga aactggaaaa cttgagtgca gaagaggaaa gtggaattcc     660

atgtgtagcg gtgaaatgcg cagagatatg gaggaacacc agtggcgaag gcgactttct     720

ggtctgtaac tgacgctgat gtgcgaaagc gtggggatca aacaggatta gataccctgg     780

tagtccacgc cgtaaacgat gagtgctaag tgttaggggg tttccgcccc ttagtgctgc     840

agctaacgca ttaagcactc cgcctgggga gtacgaccgc aaggttgaaa ctcaaaggaa     900

ttgacgggga cccgcacaag cggtggagca tgtggtttaa ttcgaagcaa cgcgaagaac     960

cttaccaaat cttgacatcc tttgaccgct ctagagatag agtcttcccc ttcgggggac    1020

aaagtgacag gtggtgcatg gttgtcgtca gctcgtgtcg tgagatgttg ggttaagtcc    1080

cgcaacgagc gcaaccctta agcttagttg ccatcattaa gttgggcact ctaagttgac    1140

tgccggtgac aaaccggagg aaggtgggga tgacgtcaaa tcatcatgcc ccttatgatt    1200

tgggctacac acgtgctaca atggacaata caaagggcag ctaaaccgcg aggtcaasca    1260

aatcccataa agttgttctc agttcggatt gtagtctgca actcgactac atgaagctgg    1320

aatcgctagt aatcgtagat cagcatgcta cggtgaatac gttcccgggt cttgtacaca    1380

ccgcccgtca caccacgaga gtttgtaaca cccgaagccg gtggagtaac catttatgga    1440

gctagccgtc gaaggtggga caaatgattg                                     1470

<210>     4
<211>     1553
<212>     RNA
<213>     Staphylococcus saprophyticus

<400>    4
tttatggaga gtttgatcct ggctcaggat gaacgctggc ggcgtgccta atacatgcaa      60

gtcgagcgaa cagataagga gcttgctcct ttgacgttag cggcggacgg gtgagtaaca     120

cgtgggtaac ctacctataa gactgggata acttcgggaa accggagcta ataccggata     180

acatttggaa ccgcatggtt ctaaagtgaa agatggtttt gctatcactt atagatggac     240

ccgcgccgta ttagctagtt ggtaaggtaa cggcttacca aggcgacgat acgtagccga     300

cctgagaggg tgatcggcca cactggaact gagacacggt ccagactcct acgggaggca     360

gcagtaggga atcttccgca atgggcgaaa gcctgacgga gcaacgccgc gtgagtgatg     420

aagggtttcg gctcgtaaaa ctctgttatt agggaagaac aaatgtgtaa gtaactgtgc     480

acgtcttgac ggtacctaat cagaaagcca cggctaacta cgtgccagca gccgcggtaa     540

tacgtaggtg gcaagcgtta tccggaatta ttgggcgtaa agcgcgcgta ggcggtttct     600

taagtctgat gtgaaagccc acggctcaac cgtggagggt cattggaaac tgggaaactt     660

gagtgcagaa gaggaaagtg gaattccatg tgtagcggtg aaatgcgcag agatatggag     720

gaacaccagt ggcgaaggcg actttctggt ctgtaactga cgctgatgtg cgaaagcgtg     780

gggatcaaac aggattagat accctggtag tccacgccgt aaacgatgag tgctaagtgt     840

tagggggttt ccgcccctta gtgctgcagc taacgcatta agcactccgc ctggggagta     900

cgaccgcaag gttgaaactc aaaggaattg acggggaccc gcacaagcgg tggagcatgt     960

ggtttaattc gaagcaacgc gaagaacctt accaaatctt gacatccttt gaaaactcta    1020

gagatagagc cttccccttc gggggacaaa gtgacaggtg gtgcatggtt gtcgtcagct    1080

cgtgtcgtga gatgttgggt taagtcccgc aacgagcgca acccttaagc ttagttgcca    1140

tcattaagtt gggcactcta ggttgactgc cggtgacaaa ccggaggaag gtggggatga    1200

cgtcaaatca tcatgcccct tatgatttgg gctacacacg tgctacaatg gacaatacaa    1260

agggcagcta aaccgcgagg tcatgcaaat cccataaagt tgttctcagt tcggattgta    1320

gtctgcaact cgactacatg aagctggaat cgctagtaat cgtagatcag catgctacgg    1380

tgaatacgtt cccgggtctt gtacacaccg cccgtcacac cacgagagtt tgtaacaccc    1440

gaagccggtg gagtaaccat ttatggagct agccgtcgaa ggtgggacaa atgattgggg    1500

tgaagtcgta acaaggtagc cgtatcggaa ggtgcggctg gatcacctcc ttt          1553


<210>    5
<211>    1468
<212>    RNA
<213>    Staphylococcus epidermidis

<400>    5
gatgaacgct ggcggcgtgc ctaatacatg caagtcgagc gaacagacga ggagcttgct      60

cctctgacgt tagcggcgga cgggtgagta acacgtggat aacctaccta taagactggg 120

ataacttcgg gaaaccggag ctaataccgg ataatatatt gaaccgcatg gttcaatagt 180

gaaagacggt tttgctgtca cttatagatg gatccgcgcc gcattagcta gttggtaagg 240

taacggctta ccaaggcaac gatgcgtagc cgacctgaga gggtgatcgg ccacactgga 300

actgagacac ggtccagact cctacgggag gcagcagtag ggaatcttcc gcaatgggcg 360

aaagcctgac ggagcaacgc cgcgtgagtg atgaaggtct tcggatcgta aaactctgtt 420

attagggaag aacaaatgtg taagtaacta tgcacgtctt gacggtacct aatcagaaag 480

ccacggctaa ctacgtgcca gcagccgcgg taatacgtag gtggcaagcg ttatccggaa 540

ttattgggcg taaagcgcgc gtaggcggtt ttttaagtct gatgtgaaag cccacggctc 600

aaccgtggag ggtcattgga aactggaaaa cttgagtgca gaagaggaaa gtggaattcc 660

atgtgtagcg gtgaaatgcg cagagatatg gaggaacacc agtggcgaag gcgactttct 720

ggtctgtaac tgacgctgat gtgcgaaagc gtggggatca aacaggatta gataccctgg 780

tagtccacgc cgtaaacgat gagtgctaag tgttaggggg tttccgcccc ttagtgctgc 840

agctaacgca ttaagcactc cgcctgggga gtacgaccgc aaggttgaaa ctcaaaggaa 900

ttgacgggga cccgcacaag cggtggagca tgtggtttaa ttcgaagcaa cgcgaagaac 960

cttaccaaat cttgacatcc tctgacccct ctagagatag agttttcccc ttcgggggac 1020

agagtgacag gtggtgcatg gttgtcgtca gctcgtgtcg tgagatgttg ggttaagtcc 1080

cgcaacgagc gcaaccctta agcttagttg ccatcattaa gttgggcact ctaagttgac 1140

tgccggtgac aaaccggagg aaggtgggga tgacgtcaaa tcatcatgcc ccttatgatt 1200

tgggctacac acgtgctaca atggacaata caaagggtag cgaaaccgcg aggtcaagca 1260

aatcccataa agttgttctc agttcggatt gtagtctgca actcgactat atgaagctgg 1320

aatcgctagt aatcgtagat cagcatgcta cggtgaatac gttcccgggt cttgtacaca 1380

ccgcccgtca caccacgaga gtttgtaaca cccgaagccg gtggagtaac catttggagc 1440

tagccgtcga aggtgggaca aatgattg 1468

<210> 6
<211> 1494
<212> RNA
<213> Staphylococcus cohnii

<400> 6
   agagtttgat cctggctcag gatgaacgct ggcggcgtgc ctaatacatg caagtcgagc 60
   gaacagataa ggagcttgct cctttgacgt tagcggcgga cgggtgagta acacgtgggt 120
   aacctaccta taagactgga ataactccgg gaaaccgggg ctaatgccgg ataacattta 180
   gaaccgcatg gttctaaagt gaaagatggt tttgctatca cttatagatg gacccgcgcc 240
   gtattagcta gttggtaagg taacggctta ccaaggcaac gatacgtagc cgacctgaga 300
   gggtgatcgg ccacactgga actgagacac ggtccagact cctacgggag gcagcagtag 360
   ggaatcttcc gcaatgggcg aaagcctgac ggagcaacgc cgcgtgagtg atgaaggtct 420
   tcggatcgta aaactctgtt attagggaag aacaaatgtg taagtaactg tgcacgtctt 480
   gacggtacct aatcagaaag ccacggctaa ctacgtgcca gcagccgcgg taatacgtag 540
   gtggcaagcg ttatccggaa ttattgggcg taaagcgcgc gtaggcggtt tcttaagtct 600
   gatgtgaaag cccacggctc aaccgtggag ggtcattgga aactgggaaa cttgagtgca 660
   gaagaggaaa gtggaattcc atgtgtagcg gtgaaatgcg cagagatatg gaggaacacc 720
   agtggcgaag gcgactttct ggtctgtaac tgacgctgat gtgcgaaagc gtggggatca 780
   aacaggatta gataccctgg tagtccacgc cgtaaacgat gagtgctaag tgttaggggg 840
   tttccgcccc ttagtgctgc agctaacgca ttaagcactc cgcctgggga gtacgaccgc 900
   aaggttgaaa ctcaaaggaa ttgacgggga cccgcacaag cggtggagca tgtggtttaa 960
   ttcgaagcaa cgcgaagaac cttaccaaat cttgacatcc tttgacaact ctagagatag 1020
   agccttcccc ttcgggggac aaagtgacag gtggtgcatg gttgtcgtca gctcgtgtcg 1080
   tgagatgttg ggttaagtcc cgcaacgagc gcaaccctta agcttagttg ccagcattaa 1140
   gttgggcact ctaagttgac tgccggtgac aaaccggagg aaggtgggga tgacgtcaaa 1200
   tcatcatgcc ccttatgatt tgggctacac acgtgctaca atggacaata caaagggcag 1260
   ctaaaccgcg aggtcatgca aatcccataa agttgttctc agttcggatt gtagtctgca 1320
   actcgactac atgaagctgg aatcgctagt aatcgtagat cagcatgcta cggtgaatac 1380
   gttcccgggt cttgtacaca ccgcccgtca caccacgaga gtttgtaaca cccgaagccg 1440
   gtggagtaac catttatgga gctagccgtc gaaggtggga caaatgattg gggt 1494

<210> 7
<211> 1475
<212> RNA
<213> Staphylococcus chromogenes

<400> 7
aggatgaacg ctggcggcgt gcctaataca tgcaagtcga gcgaactgac gaggagcttg 60

ctcctttgac gttagcggcg gacgggtgag taacacgtgg gtaacctacc tataagactg 120

gaataactcc gggaaaccgg ggctaatgcc ggataacata tcgaaccgca tggttcgata 180

gtgaaagacg gtcttgctgt cacttataga tggacccgcg ccgtattagc tagttggtga 240

ggtaacggct caccaaggca acgatacgta gccgacctga gagggtgatc ggccacactg 300

gaactgagac acggtccaga ctcctacggg aggcagcagt agggaatctt ccgcaatggg 360

cgaaagcctg acggagcaac gccgcgtgag tgatgaaggt cttcggatcg taaagctctg 420

ttgttaggga agaacaaatg tgtaagtaac tgtgcacatc ttgacggtac ctaaccagaa 480

agccacggct aactacgtgc cagcagccgc ggtaatacgt aggtggcaag cgttatccgg 540

aattattggg cgtaaagcgc gcgtaggcgg ttttttaagt ctgatgtgaa agcccacggc 600

tcaaccgtgg agggtcattg gaaactggaa aacttgagtg cagaagagga aagtggaatt 660

ccatgtgtag cggtgaaatg cgcagagata tggaggaaca ccagtggcga aggcggcttt 720

ctggtctgta actgacgctg atgtgcgaaa gcgtggggat caaacaggat tagataccct 780

ggtagtccac gccgtaaacg atgagtgcta agtgttaggg ggtttccgcc ccttagtgct 840

gcagctaacg cattaagcac tccgcctggg gagtacggtc gcaagactga aactcaaagg 900

aattgacggg gacccgcaca agcggtggag catgtggttt aattcgaagc aacgcgaaga 960

accttaccaa atcttgacat cttttgacca ctctagagat agagttttcc tcttcggagg 1020

acaaaatgac aggtggtgca tggttgtcgt cagctcgtgt cgtgagatgt tgggttaagt 1080

cccgcaacga gcgcaaccct tgagcttagt tgccatcatt aagttgggca ctctaagttg 1140

actgccggtg acaaaccgga ggaaggtggg gatgacgtca aatcatcatg ccccttatga 1200

tttgggctac acacgtgcta caatggacaa tacaaagggc agcgaaaccg cgaggtcaag 1260

caaatcccat aaagttgttc tcagttcgga ttgtagtctg caactcgact acatgaagct 1320

ggaatcgcta gtaatcgtag atcagcatgc tacggtgaat acgttcccgg gtcttgtaca 1380

caccgcccgt cacaccacga gagtttgtaa cacccgaagc cggtggagta accatttgga 1440

gctagccgtc gaaggtggga caaatgattg gggtg 1475

<210> 8
<211> 1544
<212> RNA
<213> Staphylococcus haemolyticus

<400> 8
agagtttgat tnnggctcag gatgaacgct ggcggcgtgc ctaatacatg caagtcgagc 60

gaacagacaa ggagcttgct cctttgacgt tagcggcgga cgggtgagta acacgtgggt 120

aacctaccta taagactggg ataacttcgg gaaaccggag ctaataccgg ataatatttc 180

gaaccgcatg gttcgatagt gaaagatggt tttgctatca cttatagatg gacccgcgcc 240

gtattagcta gttggtaagg taacggctta ccaaggcgac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

tcggatcgta aaactctgtt attagggaag aacatacgtg taagtaacta tgcacgtctt 480

gacggtacct aatcagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactgtaaaa cttgagtgca 660

gaagaggaaa gtggaattcc atgtgtagcg gtgaaatgcg cagagatatg gaggaacacc 720

agtggcgaag gcgactttct ggtctctaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacgaccgc 900

aaggttgaaa ctcaaaggaa ttgacgggga cccgcacaag cggtggagca tgtggtttaa 960

ttcgaagcaa gtgtaagaac cttaccaaat cttgacatcc tttgacaact ctagagatag 1020

agccttcctt cgggggacaa agtgacaggt ggtgcatggt tgtcgtcagc tcgtgtcgtg 1080

agatgttggg ttaagtcccg caacgagcgc aacccttaag cttagttgcc atcattaagt 1140

tgggcactct aagttgactg ccggtgacaa accggaggaa ggtggggatg acgtcaaatc 1200

atcatgcccc ttatgatttg ggctacacac gtgctacaat ggacaataca aagggcagcg 1260

aaaccgcgag gtcaagcaaa tcccataaag ttgttctcag ttcggattgt agtctgcaac 1320

tcgactacat gaagctggaa tcgctagtaa tcgtagatca gcatgctacg gtgaatacgt 1380

tcccgggtct tgtacacacc gcccgtcaca ccacgagagt ttgtaacacc cgaagccggt 1440

ggagtaacca tttggagcta gccgtcgaag gtgggacaaa tgattggggt gaagtcgtaa 1500

caaggtagcc gtatcggaag gtgcggctgg atcacctcct ttct 1544

<210> 9
<211> 1480
<212> RNA
<213> Staphylococcus lentus

<400> 9
aggatgaacg ctggcggcgt gcctaataca tgcaagtcga gcgaacagat gagaagcttg 60

cttctctgat gttagcggcg gacgggtgag taacacgtgg gtaacctacc tataagactg 120

ggataactcc gggaaaccgg ggctaatacc ggataatata ttgaaccgca tggttcaatg 180

ttgaaagacg gtttcggctg tcacttatag atggacccgc gccgtattag ctagttggta 240

aggtaacggc ttaccaaggc aacgatacgt agccgacctg agagggtgat cggccacact 300

ggaactgaga cacggtccag actcctacgg gaggcagcag tagggaatct tccgcaatgg 360

gcgaaagcct gacggagcaa cgccgcgtga gtgatgaagg tcttaggatc gtaaaactct 420

gttgttaggg aagaacaaat ttgttagtaa ctgaacaagt cttgacggta cctaaccaga 480

aagccacggc taactacgtg ccagcagccg cggtaatacg taggtggcaa gcgttatccg 540

gaattattgg gcgtaaagcg cgcgtaggcg gtttcttaag tctgatgtga aagcccacgg 600

ctcaaccgtg gagggtcatt ggaaactggg gaacttgagt gcagaagagg agagtggaat 660

tccatgtgta gcggtgaaat gcgcagagat atggaggaac accagtggcg aaggcggctc 720

tctggtctgt aactgacgct gatgtgcgaa agcgtgggga tcaaacagga ttagataccc 780

tggtagtcca cgccgtaaac gatgagtgct aagtgttagg gggtttccgc cccttagtgc 840

tgcagctaac gcattaagca ctccgcctgg ggagtacgac cgcaaggttg aaactcaaag 900

gaattgacgg ggacccgcac aagcggtgga gcatgtggtt taattcgaag caacgcgaag 960

aaccttacca aatcttgaca tcctttgatc gctctagaga tagagttttc cccttcgggg 1020

gacaaagtga caggtggtgc atggttgtcg tcagctcgtg tcgtgagatg ttgggttaag 1080

tcccgcaacg agcgcaaccc ttaagcttag ttgccatcat taagttgggc actctaggtt 1140

gactgccggt gacaaaccgg aggaaggtgg ggatgacgtc aaatcatcat gccccttatg 1200

atttgggcta cacacgtgct acaatggata atacaaaggg cagcgaaccc gcgaggtcaa 1260

gcaaatccca taaaattatt ctcagttcgg attgtagtct gcaactcgac tacatgaagc 1320

tggaatcgct agtaatcgta gatcagcatg ctacggtgaa tacgttcccg ggtcttgtac 1380

acaccgcccg tcacaccacg agagtttgta acacccgaag ccggtggagt aaccttttat 1440

taggagctag ccgtcgaagg tgggacagat gattggggtg 1480

<210> 10
<211> 1470
<212> RNA
<213> Staphylococcus auricularis

<400> 10
ggatgaacgc tggcggcgtg cctaatacat gcaagtcgag cgaacagata aggagcttgc 60

tcctttgacg ttagcggcgg acgggtgagt aacacgtggg taacctacct ataagactgg 120

aataactccg ggaaaccggg gctaatgccg gataacatgt tgaaccgcat ggttctacag 180

tgaaaggygg ctttgctgtc acttatagat ggacccgcgc cgtattagct agttggtgag 240

gtaacggctc accaaggcaa cgatacgtag ccgacctgag agggtgatcg gccacactgg 300

aactgagaca cggtccagac tcctacggga ggcagcagta gggaatcttc cgcaatgggc 360

gaaagcctga cggagcaacg ccgcgtgagt gatgaaggtc ttcggatcgt aaaactctgt 420

tattagggaa gaacaaatgc gtaagtaact gtgcgcgtct tgacggtacc taatcagaaa 480

gccacggyta actacgtgcc agcagccgcg gtaatacgta ggtggcaagc gttatccgga 540

attattgggc gtaaagcgcg cgtaggcggt tttttaagtc tgatgtgaaa gcccacggct 600

caaccgtgga gggtcattgg aaactgaaaa acttgagtgc agaagaggaa agtggaattc 660

catgtgtagc ggtgaaatgc gcagagatat ggaggaacac cagtggcgaa gccgactttc 720

tggtctgtaa ctgacgctga tgtgcgaaag cgtggggatc aaacaggatt agataccctg 780

gtagtcccac gccgtaaacg atgagtgcta agtgttaggg ggtttccgcc ccttagtgct 840

gtagctaacg cattaagcac tccgcctggg gagtacggcc gcaaggctta aactcaaagg 900

aattgacggg gacccgcaca agcggtggag catgtggttt aattcgaagc aacgcgaaga 960

accttaccaa atcttgacat cctttgaccg ctctagagat agagtcttcc ccttcggggg 1020

acaaagtgac aggtggtgca tggttgtcgt cagctcgtgt cgtgagatgt tgggttaagt 1080

cccgcaacga gcgcaaccct taagcttagt tgccatcatt cagttgggca ctctaagttg 1140

actgccggtg acaaaccgga ggaaggtggg gatgacgtca aatcatcatg ccccttatga 1200

tttgggctac acacgtgcta cgatggacaa tacaaagggc agcgaaaccg cgaggtcaag 1260

caaatcccat aaagttgttc tcagttcgga ttgtagtctg caactcgact acatgaagct 1320

ggaatcgcta gtaatcgtag atcagcatgc tacggtgaat acgttcccgg gtcttgtaca 1380

caccgcccgt cacaccacga gagtttgtaa cacccgaagt cggtagagta accttttgga 1440

gctagccgcc gaaggtggga caaatgattg 1470

<210> 11
<211> 1494
<212> RNA
<213> Staphylococcus equorum

<400> 11
agagtttgat cctggctcag gatgaacgct ggcggcgtgc ctaatacatg caagtcgagc 60

gaacggataa ggagcttgct cctttgaagt tagcggcgga cgggtgagta acacgtgggt 120

aacctaccta taagactgga ataacttcgg gaaaccggag ctaatgccgg ataacatttg 180

gaaccgcatg gttctaaagt aaaagatggt tttgctatca cttatagatg gacccgcgcc 240

gtattagcta gttggtaagg taacggctta ccaaggcaac gatacgtagc cgacctgaga 300

gggtgatcgg ccacactgga actgagacac ggtccagact cctacgggag gcagcagtag 360

ggaatcttcc gcaatggacg aaagtctgac ggagcaacgc cgcgtgagtg atgaaggttt 420

tcggatcgta aaactctgtt attagggaag aacaaatgtg taagtaactg tgcacatctt 480

gacggtacct aatcagaaag ccacggctaa ctacgtgcca gcagccgcgg taatacgtag 540

gtggcaagcg ttatccggaa ttattgggcg taaagcgcgc gtaggcggtt tcttaagtct 600

gatgtgaaag cccacggctc aaccgtggag ggtcattgga aactgggaaa cttgagtaca 660

gaagaggaaa gtggaattcc atgtgtagcg gtgaaatgcg cagagatatg gaggaacacc 720

agtggcgaag gcgactttct ggtctgtaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacgaccgc 900

aaggttgaaa ctcaaaggaa ttgacgggga cccgcacaag cggtggagca tgtggtttaa 960

ttcgaagcaa cgcgaagaac cttaccaaat cttgacatcc tttgaaaact ctagagatag 1020

agccttcccc ttcgggggac aaagtgacag gtggtgcatg gttgtcgtca gctcgtgtcg 1080

tgagatgttg ggttaagtcc cgcaacgagc gcaaccctta aacttagttg ccagcattta 1140

gttgggcact ctaggttgac tgccggtgac aaaccggagg aaggtgggga tgacgtcaaa 1200

tcatcatgcc ccttatgatt tgggctacac acgtgctaca atggacaata caaagggcag 1260

ctaaaccgcg aggtcatgca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac catttatgga gctagccgtc gaaggtggga caaatgattg gggt 1494

<210> 12
<211> 1474
<212> RNA
<213> Staphylococcus intermedius

<400> 12
gatgaacgct ggcggcgtgc ctaatacatg caagtcgagc gaacagataa ggagcttgct 60

cctttgacgt tagcggcgga cgggtgagta acacgtgggt aacctaccta taagactgga 120

ataactccgg gaaaccgggg ctaatgccgg ataacatgtt gaaccgcatg gttctacagt 180

gaaagacggt cttgctgtca cttatagatg gacccgcgcc gtattagcta gttggtgggg 240

taacggccta ccaaggcgac gatacgtagc cgacctgaga gggtgatcgg ccacactgga 300

actgagacac ggtccagact cctacgggag gcagcagtag ggaatcttcc gcaatgggcg 360

aaagcctgac ggagcaacgc cgcgtgagtg atgaaggtct tcggatcgta aagctctgtt 420

gttagggaag aacaaatgtg taagtaactg tgcacatctt gacggtacct aaccagaaag 480

ccacggctaa ctacgtgcca gcagccgcgg taatacgtag gtggcaagcg ttatccggaa 540

ttattgggcg taaagcgcgc gtaggcggtt ttttaagtct gatgtgaaag cccacggctc 600

aaccgtggag ggtcattgga aactggaaaa cttgagtgca gaagaggaaa gtggaattcc 660

atgtgtagcg gtgaaatgcg cagagatatg gaggaacacc agtggcgaag gcggctttct 720

ggtctgcaac tgacgctgat gtgcgaaagc gtggggatca aacaggatta gataccctgg 780

tagtccacgc cgtaaacgat gagtgctaag tgttaggggg tttccgcccc ttagtgctgc 840

agctaacgca ttaagcactc cgcctgggga gtacggtcgc aagactgaaa ctcaaaggaa 900

ttgacgggga cccgcacaag cggtggagca tgtggtttaa ttcgaagcaa cgcgaagaac 960

cttaccaaat cttgacatcc tttgaccgct ctagagatag agttttcctc ttcggaggac 1020

aaagtgacag gtggtgcatg gttgtcgtca gctcgtgtcg tgagatgttg ggttaagtcc 1080

cgcaacgagc gcaacccttg aacttagttg ccatcattca gttgggcact ctaagttgac 1140

tgccggtgac aaaccggagg aaggtgggga tgacgtcaaa tcatcatgcc ccttatgatt 1200

tgggctacac acgtgctaca atggacaata caaagggcag caaaaccgcg aggtcaagca 1260

aatcccataa agttgttctc agttcggatt gtagtctgca actcgactac atgaagctgg 1320

aatcgctagt aatcgtagat cagcatgcta cggtgaatac gttcccgggt cttgtacaca 1380

ccgcccgtca caccacgaga gtttgtaaca cccgaagccg gtggagtaac cattttggag 1440

ctagccgtcg aaggtgggac aaatgattgg ggtg 1474

<210> 13
<211> 1494
<212> RNA
<213> Staphylococcus pasteuri

<400> 13
agagtttgat cctggctcag gacgaacgct ggcggcgtgc ctaatacatg caagtcgagc 60

gaacagataa ggagcttgct cctttgacgt tagcggcgga cgggtgagta acacgtggat 120

aacctaccta taagactggg ataacttcgg gaaaccggag ctaataccgg ataagatttt 180

gaaccgcatg gttcaatagt gaaagacggc cttgctgtca cttatagatg gatccgcgcc 240

gtattagcta gttggtaagg taacggctta ccaaggcaac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

tcggatcgta aaactctgtt atcagggaag aacaaacgtg taagtaactg tgcacgtctt 480

gacggtacct gatcagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactggaaaa cttgagtgca 660

gaagaggaaa gtggaattcc atgtgtagcg gtgaaatgcg cagagatatg gaggaacacc 720

agtggcgaag gcgactttct ggtctgtaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacgaccgc 900

aaggttgaaa ctcaaaggaa ttgacgggga cccgcacaag cggtggagca tgtggtttaa 960

ttcgaagcaa cgcgaagaac cttaccaaat cttgacatcc tttgaccgct ctagagatag 1020

agttttcccc ttcgggggac aaagtgacag gtggtgcatg gttgtcgtca gctcgtgtcg 1080

tgagatgttg ggttaagtcc cgcaacgagc gcaaccctta agcttagttg ccatcattaa 1140

gttgggcact ctaagttgac tgccggtgac aaaccggagg aaggtgggga tgacgtcaaa 1200

tcatcatgcc ccttatgatt tgggctacac acgtgctaca atggacaata caaagggcag 1260

ctaaaccgcg aggtcaagca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac catttatgga gctagccgtc gaaggtggga caaatgattg gggt 1494

<210> 14
<211> 1537
<212> RNA
<213> Staphylococcus muscae

<400> 14
ttgatcctgg ctcaggatga acgctggcgg cgtgcctaat acatgcaagt cgagcgaaca 60

gacgaggtgc ttgcacctct gacgttagcg gcggacgggt gagtaacacg tgggtaacct 120

acctataaga ctggaataac ttcgggaaac cggagctaat gccggataat atattgaacc 180

gcatggttca atagtgaaag acggttttgc tgtcacttat agatggaccc gcgccgtatt 240

agctagttgg taaggtaacg gcttaccaag gcgacgatac gtagccgacc tgagagggtg 300

atcggccaca ctggaactga gacacggtcc agactcctac gggaggcagc agtagggaat 360

cttccgcaat gggcgaaagc ctgacggagc aacgccgcgt gagtgatgaa ggtcttcgga 420

tcgtaaagct ctgttattag ggaagaacaa acgtgtaagt aactgtgcac gtcttgacgg 480

tacctaatca gaaagccacg gctaactacg tgccagcagc cgcggtaata cgtaggtggc 540

aagcgttatc cggaattatt gggcgtaaag cgcgcgtagg cggtttttta agtctgatgt 600

gaaagcccac ggctcaaccg tggagggtca ttggaaactg gaaaacttga gtgcagaaga 660

ggaaagtgga attccatgtg tagcggtgaa atgcgcagag atatggagga acaccagtgg 720

cgaaggcggc tttctggtct gcaactgacg ctgatgtgcg aaagcgtggg gatcaaacag 780

gattagatac cctggtagtc cacgccgtaa acgatgagtg ctaagtgtta gggggtttcc 840

gccccttagt gctgcagcta acgcattaag cactccgcct ggggagtacg gtcgcaagac 900

tgaaactcaa aggaattgac ggggacccgc acaagcggtg gagcatgtgg tttaattcga 960

agcaacgcga agaaccttac caaatcttga catcctttga ccgcactaga gatagtgttt 1020

tcctcttcgg aggacaaagt gacaggtggt gcatggttgt cgtcagctcg tgtcgtgaga 1080

tgttgggtta agtcccgcaa cgagcgcaac ccttgagctt agttgccatc attaagttgg 1140

gcactctaag ttgactgccg gtgacaaacc ggaggaaggt ggggatgacg tcaaatcatc 1200

atgcccctta tgatttgggc tacacacgtg ctacaatgga cattacaaag ggcagcgaaa 1260

ccgcgaggtc aagcaaatcc cataaagatg ttctcagttc ggattgtagt ctgcaactcg 1320

actacatgaa gctggaatcg ctagtaatcg tagatcagca tgctacggtg aatacgttcc 1380

cgggtcttgt acacaccgcc cgtcacacca cgagagtttg taacacccga agccggtgga 1440

gtaaccattt ggagctagcc gtcgaaggtg ggacaaatga ttggggtgaa gtcgtaacaa 1500

ggtagccgta tcggaaggtg cggctggatc acctcct 1537

<210> 15

<211>	1492
<212>	RNA
<213>	Staphylococcus caprae

<400>	15
agagtttgat cctggctcag gatgaacgct ggcggcgtgc ctaatacatg caagtcgagc	60

gaacagacga ggagcttgct cctctgacgt tagcggcgga cgggtgagta acacgtggat	120

aacctaccta taagactggg ataacttcgg gaaaccggag ctaataccgg ataacatgtt	180

gaaccgcatg gttcaacagt gaaagacggt cttgctgtca cttatagatg gatccgcgcc	240

gcattagcta gttggtaagg taacggctta ccaaggcaac gatgcgtagc cgacctgaga	300

gggtgatcgg ccacactgga actgagacac ggtccagact cctacgggag gcagcagtag	360

ggaatcttcc gcaatgggcg aaagcctgac ggagcaacgc cgcgtgagtg aagaaggtct	420

tcggatcgta aaactctgtt attagggaag aacaaatgtg taagtaacta tgcacgtctt	480

gacggtacct aatcagaaag ccacggctaa ctacgtgcca gcagccgcgg taatacgtag	540

gtggcaagcg ttatccggaa ttattgggcg taaagcgcgc gtaggcggtt ttttaagtct	600

gatgtgaaag cccacggctc aaccgtggag ggtcattgga aactggaaaa cttgagtgca	660

gaagaggaaa gtggaattcc atgtgtagcg gtgaaatgcg cagagatatg gaggaacacc	720

agtggcgaag gcgactttct ggtctgtaac tgacgctgat gtgcgaaagc gtggggatca	780

aacaggatta gataccctgg tagtccacgc cgtaaacgat gagtgctaag tgttaggggg	840

tttccgcccc ttagtgctgc agctaacgca ttaagcactc cgcctgggga gtacgaccgc	900

aaggttgaaa ctcaaaggaa ttgacgggga cccgcacaag cggtggagca tgtggtttaa	960

ttcgaagcaa cgcgaagaac cttaccaaat cttgacatcc tctgacccct ctagagatag	1020

agttttcccc ttcgggggac agagtgacag gtggtgcatg gttgtcgtca gctcgtgtcg	1080

tgagatgttg ggttaagtcc cgcaacgagc gcaaccctta agcttagttg ccatcattaa	1140

gttgggcact ctaagttgac tgccggtgac aaaccggagg aaggtgggga tgacgtcaaa	1200

tcatcatgcc ccttatgatt tgggctacac acgtgctaca atggacaata caaagggcag	1260

cgaaaccgcg aggtcaagca aatcccataa agttgttctc agttcggatt gtagtctgca	1320

actcgactac atgaagctgg aatcgctagt aatcgtagat cagcatgcta cggtgaatac	1380

gttcccgggt cttgtacaca ccgcccgtca caccacgaga gtttgtaaca cccgaagccg	1440

gtggagtaac cttttggagc tagccgtcga aggtgggaca aatgattggg gt	1492

<210>	16
<211>	1494
<212>	RNA
<213>	Staphylococcus arlettae

<400>	16
   agagtttgat cctggctcag gatgaacgct ggcggcgtgc ctaatacatg caagtcgagc	60
   gaacagataa ggagcttgct cctttgacgt tagcggcgga cgggtgagta acacgtgggt 120
   aacctaccta taagactgga ataactccgg gaaaccgggg ctaatgccgg ataacattta 180
   gaaccgcatg gttctaaagt gaaagatggt tttgctatca cttatagatg gacccgcgcc 240
   gtattagcta gttggtaagg taatggctta ccaaggcaac gatacgtagc cgacctgaga 300
   gggtgatcgg ccacactgga actgagacac ggtccagact cctacgggag gcagcagtag 360
   ggaatcttcc gcaatgggcg aaagcctgac ggagcaacgc cgcgtgagtg atgaaggttt 420
   tcggctcgta aaactctgtt attagggaag aacaaacgtg taagtaactg tgcacgtctt 480
   gacggtacct aatcagaaag ccacggctaa ctacgtgcca gcagccgcgg taatacgtag 540
   gtggcaagcg ttatccggaa ttattgggcg taaagcgcgc gtaggcggtt tcttaagtct 600
   gatgtgaaag cccacggctc aaccgtggag ggtcattgga aactgggaga cttgagtgca 660
   gaagaggaaa gtggaattcc atgtgtagcg gtgaaatgcg cagagatatg gaggaacacc 720
   agtggcgaag gcgactttct ggtctgtaac tgacgctgat gtgcgaaagc gtggggatca 780
   aacaggatta gataccctgg tagtccacgc cgtaaacgat gagtgctaag tgttaggggg 840
   tttccgcccc ttagtgctgc agctaacgca ttaagcactc cgcctgggga gtacgaccgc 900
   aaggttgaaa ctcaaaggaa ttgacgggga cccgcacaag cggtggagca tgtggtttaa 960
   ttcgaagcaa cgcgaagaac cttaccaaat cttgacatcc tttgaccact ctagagatag 1020
   agttttcccc ttcgggggac aaagtgacag gtggtgcatg gttgtcgtca gctcgtgtcg 1080
   tgagatgttg ggttaagtcc cgcaacgagc gcaaccctta aacttagttg ccagcattta 1140
   gttgggcact ctaggttgac tgccggtgac aaaccggagg aaggtgggga tgacgtcaaa 1200
   tcatcatgcc ccttatgatt tgggctacac acgtgctaca atggacaata caaagggcag 1260
   ctaaaccgcg aggtcatgca aatcccataa agttgttctc agttcggatt gtagtctgca 1320
   actcgactac atgaagctgg aatcgctagt aatcgtagat cagcatgcta cggtgaatac 1380
   gttcccgggt cttgtacaca ccgcccgtca caccacgaga gtttgtaaca cccgaagccg 1440
   gtggagtaac catttatgga gctagccgtc gaaggtggga caaatgattg gggt 1494

<210> 17
<211> 1494
<212> RNA
<213> Staphylococcus kloosii

<400> 17
   agagtttgat cctggctcag gatgaacgct ggcggcgtgc ctaatacatg caagtcgagc 60
   gaacagataa ggagcttgct cctttgacgt tagcggcgga cgggtgagta acacgtgggt 120
   aacctaccta taagactgga ataacttcgg gaaaccgaag ctaatgccgg ataacatgta 180
   gaaccgcatg gttctacagt gaaagatggc cttgctatca cttatagatg gacccgcgcc 240
   gtattagcta gttggtaagg taacggctta ccaaggcaac gatacgtagc cgacctgaga 300
   gggtgatcgg ccacactgga actgagacac ggtccagact cctacgggag gcagcagtag 360
   ggaatcttcc gcaatgggcg aaagcctgac ggagcaacgc cgcgtgagtg atgaaggtct 420
   tcggatcgta aagctctgtt attagggaag aacaagtgcg taagtaactg tgcgcacctt 480
   gacggtacct aatcagaaag ccacggctaa ctacgtgcca gcagccgcgg taatacgtag 540
   gtggcaagcg ttatccggaa ttattgggcg taaagcgcgc gtaggcggtt tcttaagtct 600
   gatgtgaaag cccacggctc aaccgtggag ggtcattgga aactgggaga cttgagtgca 660
   gaagaggaaa gtggaattcc atgtgtagcg gtgaaatgcg cagagatatg gaggaacacc 720
   agtggcgaag gcgactttct ggtctgtaac tgacgctgat gtgcgaaagc gtggggatca 780
   aacaggatta gataccctgg tagtccacgc cgtaaacgat gagtgctaag tgttaggggg 840
   tttccgcccc ttagtgctgc agctaacgca ttaagcactc cgcctgggga gtacgaccgc 900
   aaggttgaaa ctcaaaggaa ttgacgggga cccgcacaag cggtggagca tgtggtttaa 960
   ttcgaagcaa cgcgaagaac cttaccaaat cttgacatcc tttgaccact ctagagacag 1020
   agctttcccc ttcgggggac aaagtgacag gtggtgcatg gttgtcgtca gctcgtgtcg 1080
   tgagatgttg ggttaagtcc cgcaacgagc gcaaccctta agcttagttg ccatcattaa 1140
   gttgggcact ctaagttgac tgccggtgac aaaccggagg aaggtgggga tgacgtcaaa 1200
   tcatcatgcc ccttatgatt tgggctacac acgtgctaca atggacaata caaagggcag 1260
   ctaaaccgcg aggtcatgca aatcccataa agttgttctc agttcggatt gtagtctgca 1320
   actcgactac atgaagctgg aatcgctagt aatcgtagat cagcatgcta cggtgaatac 1380
   gttcccgggt cttgtacaca ccgcccgtca caccacgaga gtttgtaaca cccgaagccg 1440
   gtggagtaac catttatgga gctagccgtc gaaggtggga caaatgattg gggt 1494

<210> 18
<211> 1493
<212> RNA
<213> Staphylococcus carnosus

<400> 18
agagtttgat cctggctcag gatgaacgct ggcggcgtgc ctaatacatg caagtcgagc 60

gaacagatga ggagcttgct cctctgatgt tagcggcgga cgggtgagta acacgtgggt 120

aacctaccta taagactgga ataactccgg gaaaccgggg ctaatgccgg ataatatgca 180

gaaccgcatg gttctgcaat gaaagacggt tttgctgtca cttatagatg gacccgcgcc 240

gtattagcta gttggtaagg taacggctta ccaaggcaac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

tcggatcgta aaactctgtt attagggaag aacaagtgcg taggtaacta tgcgcacctt 480

gacggtacct aatcagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactggaaaa cttgagtgca 660

gaagaggaaa gtggaattcc atgtgtagcg gtgaaatgcg cagagatatg gaggaacacc 720

agtggcgaag gcgactttct ggtctgcaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacggccgc 900

aaggctgaaa ctcaaaggaa ttgacgggga cccgcacaag cggtggagca tgtggtttaa 960

ttcgaagcaa cgcgaagaac cttaccaaat cttgacatcc tttgaccgct ctagagatag 1020

agtcttcccc ttcgggggac aaagtgacag gtggtgcatg gttgtcgtca gctcgtgtcg 1080

tgagatgttg ggttaagtcc cgcaacgagc gcaaccctta agcttagttg ccagcattaa 1140

gttgggcact ctaagttgac tgccggtgac aaaccggagg aaggtgggga tgacgtcaaa 1200

tcatcatgcc ccttatgatt tgggctacac acgtgctaca atggacagta caaagggcag 1260

cgaaaccgcg aggtcaagca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac cttttaggag ctagccgtcg aaggtgggac agatgattgg ggt 1493

<210> 19
<211> 1497
<212> RNA
<213> Staphylococcus vitulinus

<400> 19
agagtttgat cctggctcag gacgaacgct ggcggcgtgc ctaatacatg caagtcgagc 60

gaacagatga gaagcttgct tctctgatgt tagcggcgga cgggtgagta acacgtgggt 120

aacctaccta taagactggg ataactccgg gaaaccgggg ctaatacctg ataatatttt 180

gaaccgcatg gttcaatagt gaaagacggc ttcggctgtc acttatagat ggacccgcgc 240

cgtattagct agttggtaag gtaatggctt accaaggcaa cgatacgtag ccgacctgag 300

agggtgatcg gccacactgg aactgagaca cggtccagac tcctacggga ggcagcagta 360

gggaatcttc cgcaatgggc gaaagcctga cggagcaacg ccgcgtgagt gatgaaggtc 420

ttaggatcgt aaaactctgt tgttagggaa gaacaaattt gttagtaact gaacaagtct 480

tgacggtacc taaccagaaa gccacggcta actacgtgcc agcagccgcg gtaatacgta 540

ggtggcaagc gttatccgga attattgggc gtaaagcgcg cgtaggcggt ttcttaagtc 600

tgatgtgaaa gtccacggct caaccgtgga aggtcattgg aaactgggga acttgagtgc 660

agaagaggag agtggaattc catgtgtagc ggtgaaatgc gcagagatat ggaggaacac 720

cagtggcgaa ggcggctctc tggtctgtaa ctgacgctga tgtgcgaaag cgtggggatc 780

aaacaggatt agataccctg gtagtccacg ccgtaaacga tgagtgctaa gtgttagggg 840

gtttccgccc cttagtgctg cagctaacgc attaagcact ccgcctgggg agtacgaccg 900

caaggttgaa actcaaagga attgacgggg acccgcacaa gcggtggagc atgtggttta 960

attcgaagca acgcgaagaa ccttaccaaa tcttgacatc ctttgatcgc tctagagata 1020

gagttttccc cttcggggga caaagtgaca ggtggtgcat ggttgtcgtc agctcgtgtc 1080

gtgagatgtt gggttaagtc ccgcaacgag cgcaaccctt aagcttagtt gccatcatta 1140

agttgggcac tctaggttga ctgccggtga caaaccggag gaaggtgggg atgacgtcaa 1200

atcatcatgc cccttatgat ttgggctaca cacgtgctac aatggataat acaaagggca 1260

gcgaaaccgc gaggtcaagc aaatcccata aaattattct cagttcggat tgtagtctgc 1320

aactcgacta catgaagctg gaatcgctag taatcgtaga tcagcatgct acggtgaata 1380

cgttcccggg tcttgtacac accgcccgtc acaccacgag agtttgtaac acccgaagcc 1440

ggtggagtaa ccttttatta ggagctagcc gtcgaaggtg ggacagatga ttggggt 1497

<210> 20
<211> 1475
<212> RNA
<213> Staphylococcus xylosus

<400> 20
gatgaacgct ggcggcgtgc ctaatacatg caagtcgagc gaacagataa ggagcttgct 60

cctttgaagt tagcggcgga cgggtgagta acacgtgggt aacctaccta taagactggg 120

ataacttcgg gaaaccggag ctaataccgg ataacattta gaaccgcatg gttctaaagt 180

gaaagatggt tttgctatca cttatagatg gacccgcgcc gtattagcta gttggtaagg 240

taacggctta ccaaggcgac gatacgtagc cgacctgaga gggtgatcgg ccacactgga 300

actgagacac ggtccagact cctacgggag gcagcagtag ggaatcttcc gcaatgggcg 360

aaagcctgac ggagcaacgc cgcgtgagtg atgaaggttt tcggctcgta aaactctgtt 420

attagggaag aacaaatgtg taagtaactg tgcacatctt gacggtacct aatcagaaag 480

ccacggctaa ctacgtgcca gcagccgcgg taatacgtag gtggcaagcg ttatccggaa 540

ttattgggcg taaagcgcgc gtaggcggtt tcttaagtct gatgtgaaag cccacggctc 600

aaccgtggag ggtcattgga aactgggaaa cttgagtgca gaagaggaaa gtggaattcc 660

atgtgtagcg gtgaaatgcg cagagatatg gaggaacacc agtggcgaag gcgactttct 720

ggtctgtaac tgacgctgat gtgcgaaagc gtggggatca aacaggatta gataccctgg 780

tagtccacgc cgtaaacgat gagtgctaag tgttaggggg tttccgcccc ttagtgctgc 840

agctaacgca ttaagcactc cgcctgggga gtacgaccgc aaggttgaaa ctcaaaggaa 900

ttgacgggga cccgcacaag cggtggagca tgtggtttaa ttcgaagcaa cgcgaagaac 960

cttaccaaat cttgacatcc tttgaaaact ctagagatag agccttcccc ttcgggggac 1020

aaagtgacag gtggtgcatg gttgtcgtca gctcgtgtcg tgagatgttg ggttaagtcc 1080

cgcaacgagc gcaaccctta agcttagttg ccatcattaa gttgggcact ctaggttgac 1140

tgccggtgac aaaccggagg aaggtgggga tgacgtcaaa tcatcatgcc ccttatgatt 1200

tgggctacac acgtgctaca atggacaata caaagggcag ctaaaccgcg aggtcatgca 1260

aatcccataa agttgttctc agttcggatt gtagtctgca actcgactac atgaagctgg 1320

aatcgctagt aatcgtagat cagcatgcta cggtgaatac gttcccgggt cttgtacaca 1380

ccgcccgtca caccacgaga gtttgtaaca cccgaagccg gtggagtaac catttatgga 1440

gctagccgtc gaaggtggga caaatgattg gggtg 1475

<210> 21
<211> 1080
<212> RNA
<213> Unknown

<220>
<223> Staphylococcus argensis

<400> 21
ttttggtttt ggagcgcgct aactgcagtc gagcgacaga cggggagctt gctccccga 60

cgttagcggc ggacgggtga gtaacacgtg ggtaacctac ctataagact ggaataactc 120

cgggaaaccg gagctaatgc cggataacat gttggaccgc atggtcctac agtgaaagac 180

ggtcttgctg tcacttatag atggacccgc gccgtattag ctagttggta aggtaacggc 240

ttaccaaggc aacgatacgt agccgacctg agagggtgat cggccacact ggaactgaga 300

cacggtccag actcctacgg gaggcagcag tagggaatct tccgcaatgg gcgaaagcct 360

gacggagcaa cgccgcgtga gtgatgaagg ccttcgggtc gtaaaactct gttattaggg 420

aagaacaaat gtgtaagtaa ctatgcacgt cttgacggta cctaatcaga aagccacggc 480

taactacgtg ccagcagccg cggtaatacg taggtggcaa gcgttatccg gaattattgg 540

gcgtaaagcg cgcgtaggcg gtttcttaag tctgatgtga aagcccacgg ctcaaccgtg 600

gagggtcatt ggaaactggg aaacttgagt gcaggagagg aaagtggaat tccatgtgta 660

gcggtgaaat gcgcagagat atggaggaac accagtggcg aaggcgactt tctggtctgc 720

aactgacgct gatgtgcgaa agcgtgggga tcaaacagga ttagataccc tggtagtcca 780

cgccgtaaac gatgagtgct aagtgttagg gggtttccgc cccttagtgc tgcagctaac 840

gcattaagca ctccgcctgg ggagtacgac cgcaaggttg aaactcaaag gaattgacgg 900

ggacccgcac aagcggtgaa gcatgtggtt taattcgaag caaccccgaa gaaccttacc 960

aaatctttga catccttttg ccccctctag agataaaagt ttcccccttc cgggggacaa 1020

aattgacagg tgggtgcatt gtttgtcctt cacctccttg tccttgagaa tgtttgggtt 1080

1080

<210> 22
<211> 1288
<212> RNA
<213> Staphylococcus capitis

<400> 22
gcttgctcct ctgacgttag cggcggacgg gtgagtaaca cgtggataac ctacctataa 60

gactgggata acttcgggaa accggagcta ataccggata acatgttgaa ccgcatggtt 120

caacagtgaa agacggtctt gctgtcactt atagatggat ccgcgccgca ttagctagtt 180

ggtaaggtaa cggcttacca aggcaacgat gcgtagccga cctgagaggg tgatcggcca 240

cactggaact gagacacggt ccagactcct acgggaggca gcagtaggga atcttccgca 300

atgggcgaaa gcctgacgga gcaacgccgc gtgagtgaag aaggtcttcg gatcgtaaaa 360

ctctgttatt agggaagaac aaatgtgtaa gtaactatgc acgtcttgac ggtacctaat 420

cagaaagcca cggctaacta cgtgccagca gccgcggtaa tacgtaggtg gcaagcgtta 480

tccggaatta ttgggcgtaa agcgcgcgta ggcggttttt taagtctgat gtgaaagccc 540

acggctcaac cgtggagggt cattggaaac tggaaaactt gagtgcagaa gaggaaagtg 600

gaattccatg tgtagcggtg aaatgcgcag agatatggag gaacaccagt ggcgaaggcg 660

actttctggt ctgtaactga cgctgatgtg cgaaagcgtg gggatcaaac aggattagat 720

accctggtag tccacgccgt aaacgatgag tgctaagtgt tagggggttt ccgcccctta 780

gtgctgcagc taacgcatta agcactccgc ctggggagta cgaccgcaag gttgaaactc 840

aaaggaattg acggggaccc gcacaagcgg tggagcatgt ggtttaattc gaagcaacgc 900

gaagaacctt accaaatctt gacatcctct gatccctcta gagatagagt tttccccttc 960

gggggacaga gtgacaggtg gtgcatggtt gtcgtcagct cgtgtcgtga gatgttgggt 1020

taagtcccgc aacgagcgca acccttaagc ttagttgcca tcattaagtt gggcactcta 1080

agttgactgc cggtgacaaa ccggaggaag gtggggatga cgtcaaatca tcatgcccct 1140

tatgatttgg gctacacacg tgctacaatg gacaatacaa agggtagcga aaccgcgagg 1200

tcaagcaaat cccataaagt tgttctcagt tcggattgta gtctgcaact cgactacatg 1260

aagctggaat cgctagtaat cgtagatc 1288

<210>    23
<211>    1537
<212>    RNA
<213>    Unknown

<220>
<223>    Staphylococcus devriesei


<400>    23
tagagtttga tcctggctca ggatgaacgc tggcggcgtg cctaatacat gcaagtcgag      60

cgaacagata aggagcttgc tcctttgacg ttagcggcgg acgggtgagt aacacgtggg     120

taacctacct ataagactgg gataacttcg ggaaaccgga gctaataccg gataatattt     180

cgaaccgcat ggttcgatag tgaaagatgg ttttgctatc acttatagat ggatccgcgc     240

cgtattagct agttggtaag gtaacggctt accaaggcaa cgatacgtag ccgacctgag     300

agggtgatcg gccacactgg aactgagaca cggtccagac tcctacggga ggcagcagta     360

gggaatcttc cgcaatgggc gaaagcctga cggagcaacg ccgcgtgagt gatgaaggtc     420

ttcggatcgt aaaactctgt tattagggaa gaacatacgt gtaagtaact atgcacgtct     480

tgacggtacc taatcagaaa gccacggcta actacgtgcc agcagccgcg gtaatacgta     540

ggtggcaagc gttatccgga attattgggc gtaaagcgcg cgtaggcggt tttttaagtc      600

tgatgtgaaa tcccacggct caaccgtgga gggtcattgg aaactggaaa acttgagtgc     660

agaagaggaa agtggaattc catgtgtagc ggtgaaatgc gcagagatat ggaggaacac     720

cagtggcgaa ggcgactttc tggtctgtaa ctgacgctga tgtgcgaaag cgtggggatc     780

aaacaggatt agataccctg gtagtccacg ccgtaaacga tgagtgctaa gtgttagggg     840

gtttccgccc cttagtgctg cagctaacgc attaagcact ccgcctgggg agtacgaccg     900

caaggttgaa actcaaagga attgacgggg acccgcacaa gcggtggagc atgtggttta     960

attcgaagca acgcgaagaa ccttaccaaa tcttgacatc ctttgaccgc tctagagata    1020

gagttttccc cttcggggga caaagtgaca ggtggtgcat ggttgtcgtc agctcgtgtc    1080

gtgagatgtt gggttaagtc ccgcaacgag cgcaaccctt aaacttagtt gccagcattc    1140

agttgggcac tctaagttga ctgccggtga caaaccggag gaaggtgggg atgacgtcaa    1200

atcatcatgc cccttatgat ttgggctaca cacgtgctac aatggacaat acaaagggca    1260

gcgaaaccgc gaggtcaagc aaatcccata aagttgttct cagttcggat tgtagtctgc    1320

aactcgacta catgaagctg gaatcgctag taatcgtaga tcagcatgct acggtgaata    1380

cgttcccggg tcttgtacac accgcccgtc acaccacgag agtttgtaac acccgaagcc    1440

ggtggagtaa ccattttgga gctagccgtc gaaggtggga caaatgattg gggtgaagtc    1500

gtaacaaggt agccgtatcg gaaggtgcgg ctggatc                             1537

<210>     24
<211>     1528
<212>     RNA
<213>     Staphylococcus sciuri

<400>     24
gatgaacgct ggcggcgtgc ctaatacatg caagtcgagc gaacagatga gaagcttgct      60

tctctgatgt tagcggcgga cgggtgagta acacgtgggt aacctaccta taagactggg     120

ataactccgg gaaaccgggg ctaataccgg ataatatttt gaaccgcatg gttcaatagt     180

gaaagacggt ttcggctgtc acttatagat ggacccgcgc cgtattagct agttggtaag     240

gtaacggctt accaaggcga cgatacgtag ccgacctgag agggtgatcg gccacactgg     300

aactgagaca cggtccagac tcctacggga ggcagcagta gggaatcttc cgcaatgggc     360

gaaagcctga cggagcaacg ccgcgtgagt gatgaaggtc ttcggatcgt aaaactctgt     420

tgttagggaa gaacaaattt gttagtaact gaacaagtct tgacggtacc taaccagaaa     480

gccacggcta actacgtgcc agcagccgcg gtaatacgta ggtggcaagc gttatccgga     540

attattgggc gtaaagcgcg cgtaggcggt ttcttaagtc tgatgtgaaa gcccacggct     600

caaccgtgga gggtcattgg aaactgggaa acttgagtgc agaagaggag agtggaattc     660

catgtgtagc ggtgaaatgc gcagagatat ggaggaacac cagtggcgaa ggcggctctc     720

tggtctgtaa ctgacgctga tgtgcgaaag cgtggggatc aaacaggatt agataccctg     780

gtagtccacg ccgtaaacga tgagtgctaa gtgttagggg gtttccgccc cttagtgctg     840

cagctaacgc attaagcact ccgcctgggg agtacgaccg caaggttgaa actcaaagga     900

attgacgggg acccgcacaa gcggtggagc atgtggttta attcgaagca acgcgaagaa     960

ccttaccaaa tcttgacatc ctttgaccgc tctagagata gagtcttccc cttcggggga     1020

caaagtgaca ggtggtgcat ggttgtcgtc agctcgtgtc gtgagatgtt gggttaagtc    1080

ccgcaacgag cgcaaccctt aagcttagtt gccatcatta agttgggcac tctaggttga    1140

ctgccggtga caaaccggag gaaggtgggg atgacgtcaa atcatcatgc cccttatgat    1200

ttgggctaca cacgtgctac aatggataat acaaagggca gcgaatccgc gaggccaagc    1260

aaatcccata aaattattct cagttcggat tgtagtctgc aactcgacta catgaagctg    1320

gaatcgctag taatcgtaga tcagcatgct acggtgaata cgttcccggg tcttgtacac    1380

accgcccgtc acaccacgag agtttgtaac acccgaagcc ggtggagtaa ccttttagga    1440

gctagccgtc gaaggtggga caaatgattg gggtgaagtc gtaacaaggt agccgtatcg    1500

gaaggtgcgg ctggatcacc tcctttct                                       1528

<210>     25
<211>     1544
<212>     RNA
<213>     Staphylococcus hominis

<400>    25
agagtttgat nntggctcag gatgaacgct ggcggcgtgc ctaatacatg caagtcgagc    60

gaacagacga ggagcttgct cctttgacgt tagcggcgga cgggtgagta acacgtgggt    120

aacctaccta taagactggg ataacttcgg gaaaccggag ctaataccgg ataatatttc    180

gaaccgcatg gttcgatagt gaaagatggc tttgctatca cttatagatg gacctgcgcc    240

gtattagcta gttggtaagg taacggctta ccaaggcaac gatacgtagc cgacctgaga    300

gggtgatcgg ccacactgga actgagacac ggtccagact cctacgggag gcagcagtag    360

ggaatcttcc gcaatgggcg aaagcctgac ggagcaacgc cgcgtgagtg atgaaggtct    420

tcggatcgta aaactctgtt attagggaag aacaaacgtg taagtaactg tgcacgtctt    480

gacggtacct aatcagaaag ccacggctaa ctacgtgcca gcagccgcgg taatacgtag    540

gtggcaagcg ttatccggaa ttattgggcg taaagcgcgc gtaggcggtt ttttaagtct    600

gatgtgaaag cccacggctc aaccgtggag ggtcattgga aactggaaaa cttgagtgca    660

gaagaggaaa gtggaattcc atgtgtagcg gtgaaatgcg cagagatatg gaggaacacc    720

agtggcgaag gcgactttct ggtctctaac tgacgctgat gtgcgaaagc gtggggatca    780

aacaggatta gataccctgg tagtccacgc cgtaaacgat gagtgctaag tgttaggggg    840

tttccgcccc ttagtgctgc agctaacgca ttaagcactc cgcctgggga gtacgaccgc    900

aaggttgaaa ctcaaaggaa ttgacgggga cccgcacaag cggtggagca tgtggtttaa    960

ttcgaagcaa cgcgaagaac cttaccaaat cttgacatcc tttgaccctt ctagagatag    1020

aagtttcctt cgggggacaa agtgacaggt ggtgcatggt tgtcgtcagc tcgtgtcgtg    1080

agatgttggg ttaagtcccg caacgagcgc aacccttaag cttagttgcc atcattaagt    1140

tgggcactct aagttgactg ccggtgacaa accggaggaa ggtggggatg acgtcaaatc    1200

atcatgcccc ttatgatttg ggctacacac gtgctacaat ggacaataca aagggcagcg    1260

aaaccgcgag gtcaagcaaa tcccataaag ttgttctcag ttcggattgt agtctgcaac    1320

tcgactacat gaagctggaa tcgctagtaa tcgtagatca gcatgctacg gtgaatacgt    1380

tcccgggtct tgtacacacc gcccgtcaca ccacgagagt ttgtaacacc cgaagccggt    1440

ggagtaacca tttggagcta gccgtcgaag gtgggacaaa tgattggggt gaagtcgtaa    1500

caaggtagcc gtatcggaag gtgcggctgg atcacctcct ttct    1544

<210>    26
<211>    1477
<212>    RNA
<213>    Unknown

<220>
<223>    Staphylococcus microti

<400>    26
ggatgaacgc tggcggcgtg cctaatacat gcaagtcgag cgaacagacg aggtgcttgc      60

acctctgacg ttagcggcgg acgggtgagt aacacgtggg taacctacct ataagactgg     120

gataactccg ggaaaccggg gctaataccg gataacatat tgaaccgcat ggttcaatag     180

tgaaagacgg ttttgctgtc acttatagat ggacccgcgc cgtattagct agttggtgag     240

gtaacggctc accaaggcga cgatacgtag ccgacctgag agggtgatcg gccacactgg     300

aactgagaca cggtccagac tcctacggga ggcagcagta gggaatcttc cgcaatgggc     360

gaaagcctga cggagcaacg ccgcgtgagt gatgaaggtc ttcggatcgt aaagctctgt     420

tattagggaa gaacaaacgt gtaagtaact gtgcacgtct tgacggtacc taatcagaaa     480

gccacggcta actacgtgcc agcagccgcg gtaatacgta ggtggcaagc gttatccgga     540

attattgggc gtaaagcgcg cgtaggcggt tttttaagtc tgatgtgaaa gcccacggct     600

caaccgtgga gggtcattgg aaactggaaa acttgagtgc agaagaggaa agtggaattc     660

catgtgtagc ggtgaaatgc gcagagatat ggaggaacac cagtggcgaa ggcggctttc     720

tggtctgcaa ctgacgctga tgtgcgaaag cgtggggatc aaacaggatt agataccctg     780

gtagtccacg ccgtaaacga tgagtgctaa gtgttagggg gtttccgccc cttagtgctg     840

cagctaacgc attaagcact ccgcctgggg agtacggtcg caagactgaa actcaaagga     900

attgacgggg acccgcacaa gcggtggagc atgtggttta attcgaagca acgcgaagaa     960

ccttaccaaa tcttgacatc ctttgaccgc actagagata gtgttttcct cttcggagga    1020

caaagtgaca ggtggtgcat ggttgtcgtc agctcgtgtc gtgagatgtt gggttaagtc    1080

ccgcaacgag cgcaaccctt gagcttagtt gccatcatta agttgggcac tctaagctga    1140

ctgccggtga caaaccggag gaaggtgggg atgacgtcaa atcatcatgc cccttatgat    1200

ttgggctaca cacgtgctac aatggacatt acaaagggca gcaaaaccgc gaggtcaagc    1260

aaatcccata aagatgttct cagttcggat tgtagtctgc aactcgacta catgaagctg    1320

gaatcgctag taatcgtaga tcagcatgct acggtgaata cgttcccggg tcttgtacac    1380

accgcccgtc acaccacgag agtttgtaac acccgaagcc ggtggagtaa ccatttggag    1440

ctagccgtcg aaggtgggac aaatgattgg ggtgaag                             1477

<210>    27
<211>    1493
<212>    RNA
<213>    Unknown

<220>
<223>    Staphylococcus piscifermentans

<400>    27

agagtttgat cctggctcag gatgaacgct ggcggcgtgc ctaatacatg caagtcgagc 60

gaacagacga ggagcttgct cctctgacgt tagcggcgga cgggtgagta acacgtgggt 120

aacctaccta taagactgga ataactccgg gaaaccgggg ctaatgccgg ataatatgcg 180

gaaccgcatg gttccgcaat gaaagacggt tttgctgtca cttatagatg gacccgcgcc 240

gtattagcta gttggtaagg taacggctta ccaaggcaac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

tcggatcgta aaactctgtt attagggaag aacaagtgcg taggtaacta tgcgcacctt 480

gacggtacct aatcagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactggaaaa cttgagtgca 660

gaagaggaaa gtggaattcc atgtgtagcg gtgaaatgcg cagagatatg gaggaacacc 720

agtggcgaag gcgactttct ggtctgcaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacggccgc 900

aaggctgaaa ctcaaaggaa ttgacgggga cccgcacaag cggtggagca tgtggtttaa 960

ttcgaagcaa cgcgaagaac cttaccaaat cttgacatcc tttgaccgct ctagagatag 1020

agtcttcccc ttcgggggac aaagtgacag gtggtgcatg gttgtcgtca gctcgtgtcg 1080

tgagatgttg ggttaagtcc cgcaacgagc gcaaccctta agcttagttg ccagcattaa 1140

gttgggcact ctaagttgac tgccggtgac aaaccggagg aaggtgggga tgacgtcaaa 1200

tcatcatgcc ccttatgatt tgggctacac acgtgctaca atggacaata caaagggcag 1260

cgaaaccgcg aggtcaagca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac cttttaggag ctagccgtcg aaggtgggac agatgattgg ggt 1493

<210> 28
<211> 819
<212> RNA
<213> Unknown

<220>
<223> Staphylococcus schweitzeri

<400> 28
gcctgacgga gcacgccgcg tgagtgatga aggtcttcgg atcgtaaaac tctgttatta 60

gggaagaaca tatgtgtaag taactgtgca catcttgacg gtacctaatc agaaagccac 120

ggctaactac gtgccagcag ccgcggtaat acgtaggtgg caagcgttat ccggaattat 180

tgggcgtaaa gcgcgcgtag gcggtttttt aagtctgatg tgaaagccca cggctcaacc 240

gtggagggtc attggaaact ggaaaacttg agtgcagaag aggaaagtgg aattccatgt 300

gtagcggtga aatgcgcaga gatatggagg aacaccagtg gcgaaggcga ctttctggtc 360

tgtaactgac gctgatgtgc gaaagcgtgg ggatcaaaca ggattagata ccctggtagt 420

ccacgccgta aacgatgagt gctaagtgtt agggggtttc cgccccttag tgctgcagct 480

aacgcattaa gcactccgcc tggggagtac gaccgcaagg ttgaaactca aaggaattga 540

cggggacccg cacaagcggt ggagcatgtg gtttaattcg aagcaacgcg aagaacctta 600

ccaaatcttg acatcctttg acaactctag agatagagct ttcctcttcg ggggacaaag 660

tgacaggtgg tgcatggttg tcgtcagctc gtgtcgtgag atgttgggtt aagtcccgca 720

acgagcgcaa cccttaagct tagttgccat cattaagttg ggcactctaa gttgactgcc 780

ggtgacaaac cggaggaagg tggggatgac gtcaaatca 819

<210> 29
<211> 1476
<212> RNA
<213> Staphylococcus simulans

<400> 29
aggatgaacg ctggcggcgt gcctaataca tgcaagtcga gcgaacagac gaggagcttg 60

ctcctctgac gttagcggcg gacgggtgag taacacgtgg gtaacctacc tataagactg 120

ggataactcc gggaaaccgg ggctaatacc ggataacaca tgaaaccgca tggtttcatg 180

atgaaagacg gttttgctgt cacttataga tggacccgcg gcgtattagc tagttggtaa 240

ggtaacggct taccaaggca acgatacgta gccgacctga gagggtgatc ggccacactg 300

gaactgagac acggtccaga ctcctacggg aggcagcagt agggaatctt ccgcaatggg 360

cgaaagcctg acggagcaac gccgcgtgag tgatgaaggt cttcggatcg taaaactctg 420

ttattaggga agaacaagga tgtaagtaac tatgcatccc ttgacggtac ctaatcagaa 480

agccacggct aactacgtgc cagcagccgc ggtaatacgt aggtggcaag cgttatccgg 540

aattattggg cgtaaagcgc gcgtaggcgg ttttttaagt ctgatgtgaa agcccacggc 600

tcaaccgtgg agggtcattg gaaactggaa aacttgagtg cagaagagga aagtggaatt 660

ccatgtgtag cggtgaaatg cgcagagata tggaggaaca ccagtggcga aggcgacttt 720

ctggtctgca actgacgctg atgtgcgaaa gcgtggggat caaacaggat tagataccct 780

ggtagtccac gccgtaaacg atgagtgcta agtgttaggg ggtttccgcc ccttagtgct 840

gcagctaacg cattaagcac tccgcctggg gagtacggcc gcaaggctga aactcaaagg 900

aattgacggg gacccgcaca agcggtggag catgtggttt aattcgaagc aacgcgaaga 960

accttaccaa atcttgacat cctttgacaa ctctagagat agagctttcc ccttcggggg 1020

acaaagtgac aggtggtgca tggttgtcgt cagctcgtgt cgtgagatgt tgggttaagt 1080

cccgcaacga gcgcaaccct taagcttagt tgccagcatt aagttgggca ctctaagttg 1140

actgccggtg acaaaccgga ggaaggtggg gatgacgtca aatcatcatg ccccttatga 1200

tttgggctac acacgtgcta caatggacgg tacaaagggc agcgaacccg cgaggtcaag 1260

caaatcccat aaagccgttc tcagttcgga ttgtagtctg caactcgact acatgaagct 1320

ggaatcgcta gtaatcgtag atcagcatgc tacggtgaat acgttcccgg gtcttgtaca 1380

caccgcccgt cacaccacga gagtttgtaa cacccgaagc cggtggagta accttttagg 1440

aactagccgt cgaaggtggg acaaatgatt ggggtg 1476

<210> 30
<211> 1548
<212> RNA
<213> Unknown

<220>
<223> Staphylococcus succinus

<400> 30
tggagagttt gatcctggct caggatgaac gctggcggcg tgcctaatac atgcaagtcg 60

agcgaacgga taaggagctt gctcctttga agttagcggc ggacgggtga gtaacacgtg 120

ggtaacctac ctataagact ggaataactt cgggaaaccg gagctaatgc cggataacat 180

atagaaccgc atggttctat agtgaaagat ggttttgcta tcacttatag atggacccgc 240

gccgtattag ctagttggta aggtaatggc ttaccaaggc gacgatacgt agccgacctg 300

agagggtgat cggccacact ggaactgaga cacggtccag actcctacgg gaggcagcag 360

tagggaatct tccgcaatgg gcgaaagcct gacggagcaa cgccgcgtga gtgatgaagg 420

ttttcggatc gtaaaactct gttattaggg aagaacaaat gcgtaagtaa ctgtgcgcat 480

cttgacggta cctaatcaga aagccacggc taactacgtg ccagcagccg cggtaatacg 540

taggtggcaa gcgttatccg gaattattgg gcgtaaagcg cgcgtaggcg gtttcttaag 600

tctgatgtga aagcccacgg ctcaaccgtg gagggtcatt ggaaactggg aaacttgagt 660

gcagaagagg aaagtggaat tccatgtgta gcggtgaaat gcgcagagat atggaggaac 720

accagtggcg aaggcgactt tctggtctgt aactgacgct gatgtgcgaa agcgtgggga 780

tcaaacagga ttagataccc tggtagtcca cgccgtaaac gatgagtgct aagtgttagg 840

gggtttccgc cccttagtgc tgcagctaac gcattaagca ctccgcctgg ggagtacgac 900

cgcaaggttg aaactcaaag gaattgacgg ggacccgcac aagcggtgga gcatgtggtt 960

taattcgaag caacgcgaag aaccttacca aatcttgaca tcctttgaaa actctagaga 1020

tagagccttc cccttcgggg gacaaagtga caggtggtgc atggttgtcg tcagctcgtg 1080

tcgtgagatg ttgggttaag tcccgcaacg agcgcaaccc ttaagcttag ttgccatcat 1140

taagttgggc actctaggtt gactgccggt gacaaaccgg aggaaggtgg ggatgacgtc 1200

aaatcatcat gccccttatg atttgggcta cacacgtgct acaatggaca atacaaaggg 1260

cagctaaacc gcgaggtcat gcaaatccca taaagttgtt ctcagttcgg attgtagtct 1320

gcaactcgac tacatgaagc tggaatcgct agtaatcgta gatcagcatg ctacggtgaa 1380

tacgttcccg ggtcttgtac acaccgcccg tcacaccacg agagtttgta acacccgaag 1440

ccggtggagt aaccatttat ggagctagcc gtcgaaggtg ggacaaatga ttggggtgaa 1500

gtcgtaacaa ggtagccgta tcggaaggtg cggctggatc acctcctt 1548

<210> 31
<211> 1492
<212> RNA
<213> Staphylococcus lugdunensis

<400> 31
agagtttgat cctggctcag gatgaacgct ggcggcgtgc ctaatacatg caagtcgagc 60

gaacagataa ggagcttgct cctttgacgt tagcggcgga cgggtgagta acacgtgggt 120

aacctaccta taagactggg acaacttcgg gaaaccggag ctaataccgg ataatatgtt 180

gaaccgcatg gttcaatagt gaaagatggt tttgctatca cttatagatg gacccgcgcc 240

gtattagcta gttggtgagg taacggctca ccaaggcaac gatacgtagc cgacctgaga 300

gggtgatcgg ccacactgga actgagacac ggtccagact cctacgggag gcagcagtag 360

ggaatcttcc gcaatgggcg aaagcctgac ggagcaacgc cgcgtgagtg atgaaggtct 420

taggatcgta aaactctgtt attagggaag aacaaacgtg taagtaactg tgcacgtctt 480

gacggtacct aatcagaaag ccacggctaa ctacgtgcca gcagccgcgg taatacgtag 540

gtggcaagcg ttatccggaa ttattgggcg taaagcgcgc gtaggcggtt ttttaagtct 600

gatgtgaaag cccacggctc aaccgtggag ggtcattgga aactggaaaa cttgagtgca 660

gaagaggaaa gtggaattcc atgtgtagcg gtgaaatgcg cagagatatg gaggaacacc 720

agtggcgaag gcgactttct ggtctgtaac tgacgctgat gtgcgaaagc gtggggatca 780

aacaggatta gataccctgg tagtccacgc cgtaaacgat gagtgctaag tgttaggggg 840

tttccgcccc ttagtgctgc agctaacgca ttaagcactc cgcctgggga gtacgaccgc 900

aaggttgaaa ctcaaaggaa ttgacgggga cccgcacaag cggtggagca tgtggtttaa 960

ttcgaagcaa cgcgaagaac cttaccaaat cttgacatcc tttgaccgct ctagagatag 1020

agtcttcccc ttcgggggac aaagtgacag gtggtgcatg gttgtcgtca gctcgtgtcg 1080

tgagatgttg ggttaagtcc cgcaacgagc gcaaccctta agcttagttg ccatcattta 1140

gttgggcact ctaagttgac tgccggtgac aaaccggagg aaggtgggga tgacgtcaaa 1200

tcatcatgcc ccttatgatt tgggctacac acgtgctaca atggacaata caaagggcag 1260

cgaaaccgcg aggtcaagca aatcccataa agttgttctc agttcggatt gtagtctgca 1320

actcgactac atgaagctgg aatcgctagt aatcgtagat cagcatgcta cggtgaatac 1380

gttcccgggt cttgtacaca ccgcccgtca caccacgaga gtttgtaaca cccgaagccg 1440

gtggagtaac catttggagc tagccgtcga aggtgggaca aatgattggg gt 1492